# EUROPÄISCHE PATENTSCHRIFT

(11) **EP 2 783 677 B1**
(45) Veröffentlichungstag und Bekanntmachung des Hinweises auf die Patenterteilung: **04.05.2022**
(21) Anmeldenummer: 14157418.6
(22) Anmeldetag: 03.03.2014
(51) Int. Cl.: A61K 8/37, A61K 8/41, A61Q 5/12, A61Q 5/00, A61Q 5/02, A61Q 5/06, A61Q 19/00, A61Q 19/10

(54) **Formulierung enthaltend Esterquats basierend auf Isopropanolamin**
Formulation comprising ester quats based on isopropanolamine
Formulation contenant des esterquats à base d'isopropanolamine

(30) Priorität: 22.03.2013 DE 102013205092
(43) Veröffentlichungstag der Anmeldung: 01.10.2014
(73) Patentinhaber: Evonik Operations GmbH, 45128 Essen (DE)
(72) Erfinder: Köhle, Hans-Jürgen, 63533 Mainhausen (DE); Seidel, Kurt, 63637 Jossgrund (DE); Schwab, Peter, 45133 Essen (DE); Westerholt, Ursula, 45138 Essen (DE)
(74) Vertreter: Evonik Patent Association

(56) Entgegenhaltungen:
- EP-A2- 0 293 953
- WO-A1-99/35223
- DE-A1- 2 430 140
- DE-A1-102010 029 606

## Beschreibung

### Gebiet der Erfindung

Gegenstand der Erfindung sind kosmetische Formulierungen enthaltend bestimmte Esterquats basierend auf Isopropanolamin sowie die Verwendung dieser Esterquats in der Kosmetik.

### Stand der Technik

Die DE3608093 beschreibt quartäre Ammoniumverbindungen enthaltend zwei 2-Acyloxyalkylgruppen, deren Acylgruppen sich von gesättigten oder ungesättigten Carbonsäuren mit 12 bis 22 Kohlenstoffatome ableiten, unter anderem auch Dimethyl-di-(oleoyl-oxyisopropyl-)ammoniummethosulfat, und die Verwendung solcher Substanzen in textilweichmachenden Formulierungen.

Die DE3877422 beschreibt ähnliche quartäre Ammoniumverbindungen, deren entsprechende Acylgruppen maximal 17 Kohlenstoffatome enthalten, unter anderem Dimethyl-di-(palmitoyl-oxyisopropyl-)ammoniumchlorid, und die Verwendung solcher Substanzen in textilweichmachenden Formulierungen, erwähnt aber auch deren Eignung für Haarkonditioniermittel.

Bisher erhältlich Esterquats, welche sich zur Verwendung in der Haarkonditionierung eignen, sind feste Substanzen, welche erst durch den Einsatz von Lösungsmitteln in eine formulierbare Form gebracht werde, wodurch festgelegt wird, welches Lösungsmittel in der Endformulierung enthalten sein wird. Dies schränkt die Freiheitsgrade der Formulierungsmöglichkeiten ein.

Aufgabe der Erfindung war es, eine Zusammensetzung bereitzustellen, die hervorragende konditionierende Effekte auf keratinische Fasern ausübt.

### Beschreibung der Erfindung

Überraschenderweise wurde gefunden, dass die im Folgenden beschriebenen Formulierungen die der Erfindung gestellte Aufgabe zu lösen vermögen.

Gegenstand der vorliegenden Erfindung sind daher kosmetische Formulierungen, enthaltend
0,2 bis 25 Gew.-%, bevorzugt 0,5 bis 15 Gew.-%, insbesondere 1 bis 10 Gew.-%, mindestens einer Verbindung der allgemeinen Formel I) 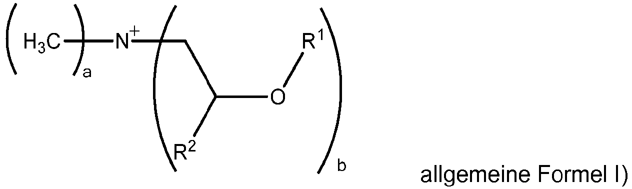
mit R¹ ein Acylrest einer mindestens einfach ungesättigten Fettsäure mit einer Kettenlänge von 18 bis 24 Kohlenstoffatomen oder der Acylrest der Isostearinsäure oder Rizinolsäure,
mit R² ein Alkylrest mit 1 bis 6 Kohlenstoffatomen, bevorzugt Methyl, Ethyl, Propyl, Isopropyl, besonders bevorzugt Methyl,
wobei a = 1 bis 3 und b = 1 bis 3,
mit der Maßgabe, dass a + b =4 und
40 bis 99,8 Gew.-%, bevorzugt 50 bis 99,5 Gew.-%, insbesondere 60 bis 99 Gew.-%, Wasser, und gegebenenfalls
0 bis 8 Gew.-% eine Verbindung der allgemeinen Formel Ia) 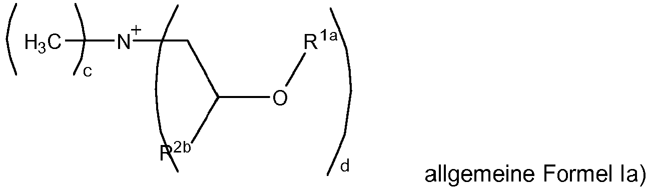
mit R^{1a} ein Acylrest einer anderen Carbonsäure als für R¹ definiert und mit R^{2b} ein Alkylrest mit 1 bis 6 Kohlenstoffatomen, bevorzugt Methyl, Ethyl, Propyl, Isopropyl, besonders bevorzugt Methyl,
wobei c = 1 bis 3 und d = 1 bis 3,
mit der Maßgabe, dass c + d = 4,
wobei sich die Gewichtsprozente auf die Gesamtformulierung beziehen, dadurch gekennzeichnet, dass
die Verbindungen der allgemeinen Formel I) mindestens 350 Gew.-%, bezogen auf alle in der Formulierung enthaltene Verbindungen der allgemeinen Formel I) und Ia) ausmachen
und dadurch gekennzeichnet, dass
die Verbindungen der allgemeinen Formel I)
mit R¹ ein Acylrest einer mindestens einfach ungesättigten Fettsäure mit einer Kettenlänge von 18 bis 24 Kohlenstoffatomen ausgewählt aus den Acylresten der Säuren aus der Gruppe Ölsäure, Linolsäure, Alpha-Linolensäure und Gamma-Linolensäure, oder ein Acylrest der Isostearinsäure oder der Rizinolsäure mindestens 50 Gew.-%, bezogen auf alle in der Formulierung enthaltene Verbindungen der allgemeinen Formel I) und Ia) ausmachen.

Ein weiterer Gegenstand der Erfindung ist Verwendung einer Verbindung der allgemeinen Formel I) 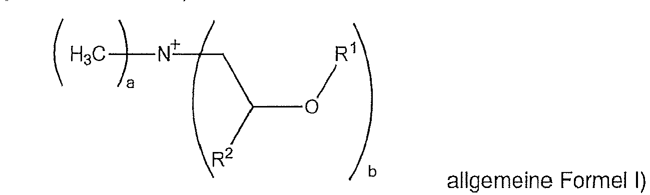
mit R¹ ein Acylrest einer mindestens einfach ungesättigten Fettsäure mit einer Kettenlänge von 18 bis 24 Kohlenstoffatomen oder der Acylrest der Isostearinsäure oder Rizinolsäure,
mit R² ein Alkylrest mit 1 bis 6 Kohlenstoffatomen, bevorzugt Methyl, Ethyl, Propyl, Isopropyl, besonders bevorzugt Methyl,,
wobei a = 1 bis 3 und b = 1 bis 3,
mit der Maßgabe, dass a + b =4
zur kosmetischen Behandlung von keratinischen Fasern, insbesondere Haaren, wobei menschliches Haar bevorzugt ist.

Ein weiterer Gegenstand der Erfindung ist eine Verbindung der allgemeinen Formel I) 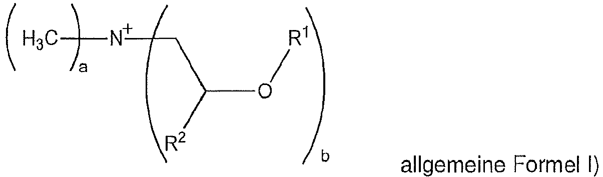
mit R¹ ein Acylrest der Isostearinsäure,
mit R² ein Alkylrest mit 1 bis 6 Kohlenstoffatomen, bevorzugt Methyl, Ethyl, Propyl, Isopropyl, besonders bevorzugt Methyl,
wobei a = 1 bis 3 und b = 1 bis 3
mit der Maßgabe, dass a + b =4.

Ein Vorteil der vorliegenden Erfindung ist es, dass die eingesetzten Esterquats bei Raumtemperatur flüssig sind und somit ohne den Einsatz von Lösungsmittel in eine Endkundenformulierung einfach eingearbeitet werden können, wodurch in dieser nicht zwangsläufig solch ein Lösungsmittel enthalten sein muss.

Noch ein Vorteil der vorliegenden Erfindung ist es, dass der Glanz der behandelten keratinischen Faserngesteigert wird.

Ein weiterer Vorteil der vorliegenden Erfindung ist, dass die eingesetzten Verbindungen bereits bei geringen Einsatzmengen eine gute Wirkung entfalten.

Ein weiterer Vorteil ist, dass die eingesetzten Verbindungen in ökologischer Hinsicht wenig belastend sind.

Noch ein Vorteil ist es, dass die eingesetzten Verbindungen auf keratinischen Fasern einen verbesserten konditionierenden Effekt bei längeren Abspülzeiten zeigen als bisher bekannte quartäre Esterverbindungen.

Noch ein Vorteil der vorliegenden Erfindung ist es, dass sie eine erhöhte Hydrolysestabilität in der Formulierung aufweisen.

Noch ein Vorteil der vorliegenden Erfindung ist es, dass sie nicht auskristallisieren Noch ein Vorteil der vorliegenden Erfindung ist es, dass sie in geringeren Einsatzkonzentrationen wirksam sind

Noch ein Vorteil der vorliegenden Erfindung ist es, dass sie Haarfärbemittel vor dem Auswaschen schützen

Noch ein Vorteil der vorliegenden Erfindung ist es, dass sie keratinische Fasern gegenüber thermisch induzierten Schäden schützen

Noch ein Vorteil der vorliegenden Erfindung ist es, dass sie die Kammkräfte am nassen und am trockenen Haar reduzieren

Noch ein Vorteil der vorliegenden Erfindung ist es, dass sie methanolfrei hergestellt werden können.

Noch ein Vorteil der vorliegenden Erfindung ist es, dass sie besonders wirtschaftlich ist.

Alle angegebenen Prozent (%) sind, wenn nicht anders angegeben, Massenprozent.

Die im Rahmen der Erfindung eingesetzten quaternierten Fettsäureisopropanolaminester-Salze lassen sich nach einschlägigen Methoden der präparativen organischen Chemie herstellen. Üblicherweise beruht die Herstellung von Esterquats auf einem mehrstufigen Prozess, bei dem zunächst durch Umsetzung eines Alkanolamins mit Carbonsäuren oder entsprechenden Derivaten das veresterte Alkanolamin hergestellt wird, das dann im Anschluss mit einem geeigneten Reagens quaterniert wird. Im Zusammenhang mit der vorliegenden Erfindung werden als AlkanolaminDi-Methyl-Mono-, Methyl-Di- oder Tri-isopropanol-Amin oder Mischungen davon, insbesondere Methyl-Di-isopropanolamin, eingesetzt

In Zusammenhang für geeignete Herstellverfahren sei auf die EP0483195 verwiesen, nach der man Trialkanolamin in Gegenwart von unterphosphoriger Säure mit Fettsäuren partiell verestert, Luft durchleitet und anschließend mit Dimethylsulfat oder Ethylenoxidquaterniert. Die dort aufgeführten Verbindungen dienen als Weichmacher für Textilien. In der DE4308794 wird die Herstellung fester Esterquats beschrieben, indem die Quaternisierung der Triethanolaminester in Gegenwart geeigneter Dispergiermittel durchgeführt wird. Übersichten zu dieser Thematik lassen sich zum Beispiel unter R. Puchta et al. in Tens. Surf. Det., 30, 186 (1993), M. Brock in Tens. Surf. Det., 30, 394 (1993), R. Lagerman et al. in J. Am. Chem. Soc., 71, 97 (1994) oder unter I. Shapiro in Cosm. Toil., 109, 77 (1994) nachlesen.

Für den Fall, dass b>1 ist, können die Reste R¹ gleich oder ungleich sein.

R¹ als Acylrest einer mindestens einfach ungesättigten Fettsäure mit einer Kettenlänge von 18 bis 24 Kohlenstoffatomen kann eine oder mehr, beispielsweise zwei oder drei Doppelbindungen enthalten.

Erfindungsgemäß bevorzugte Formulierungen sind dadurch gekennzeichnet, dass R¹ als Acylrest einer mindestens einfach ungesättigten Fettsäure mit einer Kettenlänge von 18 bis 24 Kohlenstoffatomen ausgewählt ist aus den Acylresten der Säuren aus der Gruppe umfassend

Ölsäure, Elaidinsäure, Vaccensäure, Gadoleinsäure, Icosensäure, Cetoleinsäure, Erucasäure, Nervonsäure, Linolsäure, Alpha-Linolensäure, Gamma-Linolensäure, Calendulasäure, Punicinsäure, Alpha-Elaeostearinsäure, Beta-Elaeostearinsäure, Arachidonsäure, Timnodonsäure, Clupanodonsäure, und Cervonsäure, wobei Ölsäure besonders bevorzugt ist.

Es können erfindungsgemäß auch Mischungen dieser Carbonsäuren eingesetzt werden.

Bevorzugte Formulierungen enthalten mindestens eine Verbindung der allgemeinen Formel I) mit a = b = 2.

Eine erfindungsgemäß besonders bevorzugte Formulierung ist dadurch gekennzeichnet, dass R¹ der Acylrest der Ölsäure und a = b = 2 ist.

In der erfindungsgemäßen Formulierung können neben einer Verbindung der allgemeinen Formel I) weitere Verbindungen enthalten sein, die bis auf den Rest R¹ der Verbindung der allgemeinen Formel I) entsprechen, das heißt der analoge Rest R^{1a} ist der Acylrest einer anderen Carbonsäure, insbesondere einer anderen Fettsäure.

Die Formulierungen enthalten somit bevorzugt eine Mischung mindestens einer Verbindung der allgemeinen Formel I) und mindestens einer Verbindung der allgemeinen Formel Ia), wie sie sich beispielsweise bei dem Einsatz von technischen Fettsäureschnitten ergibt, welche längere oder kürzere Acylreste aufweisen, als für R¹ oben definiert.

Die Verbindungen der allgemeinen Formel I) machen mindestens 50 Gew.-%, besonders bevorzugt mindestens 75 Gew.-%, bezogen auf alle Verbindungen der allgemeinen Formel I) und Ia) aus.

Ganz besonders bevorzugt sind Mischungen, die erhalten werden, wenn als Mischung Pflanzenmischöle mit einer C-Kettenverteilung eingesetzt werden, für die gilt: Kettenlänge von R¹ bzw. R^{1a} Anteil bezogen auf Gesamtmischung (' = Anzahl der Doppelbindung(en))

| | |
|---|---|
| <C 16 | 0 - 2 Gew.-% |
| C 16 | 4 - 7 Gew.-% |
| C 16' | 0 - 2 Gew.-% |
| C 18 | 0 - 4 Gew.-% |
| C 18' | 55 - 65 Gew.-% |
| C 18" | 15 - 25 Gew.-% |
| C 18"' | 6 - 12 Gew.-% |
| >C 18 | 0 - 4 Gew.-% |

Bevorzugte Formulierungen dieser Ausführungsform enthalten Verbindungen der allgemeinen Formel I) bzw. Ia) mit a = b = c = d = 2.

Ein weiterer Gegenstand der Erfindung ist eine Verbindung der allgemeinen Formel I) 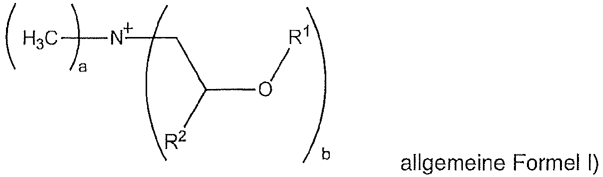
mit R¹ ein Acylrest der Isostearinsäure,
mit R² ein Alkylrest mit 1 bis 6 Kohlenstoffatomen, bevorzugt Methyl, Ethyl, Propyl, Isopropyl, besonders bevorzugt Methyl,
wobei a = 1 bis 3 und b = 1 bis 3
mit der Maßgabe, dass a + b =4.

Erfindungsgemäß bevorzugte Verbindungen der allgemeinen Formel I) sind solche mit a = b = 2, insbesondere bevorzugt mit R2 = Methyl.

Bevorzugte erfindungsgemäße Formulierungen sind, dadurch gekennzeichnet, dass sie keine Fettsäuren oder Fettsäuresalze enthalten.

Es hat sich als vorteilhaft erwiesen, wenn die erfindungsgemäßen Formulierungen zusätzlich 0,5 bis 20 Gew.-%, bevorzugt 1 bis 10 Gew.-%, insbesondere 2 bis 7 Gew.-%, mindestens eines Fettalkohols, enthalten, wobei sich die Gewichtsprozente auf die Gesamtformulierung beziehen.

Als Fettalkohol wird in diesem Zusammenhang bevorzugt ein unverzweigter oder verzweigter Monoalkohol mit einer Alkylgruppevon 8 bis 30 C-Atomen verstanden, der auch ungesättigt sein kann. Bevorzugte Fettalkohole sind Octanol, Decanol, Laurylalkohol, lsolaurylalkohol, Anteisolaurylalkohol, Myristylalkohol, lsomyristylalkohol, Cetylalkohol, Palmoleylalkohol, Stearylalkohol,lsostearylalkohol, Anteisostearylalkohol, Eicosanol, Petroselinylalkohol, Guerbetalkohol, Arachylalkohol, Gadoleylalkohol, Behenylalkohol, Erucylalkohol, Heptacosanol, Octacosanol, und Melissylalkohol sowie Mischungen davon, insbesondere technische Mischungen, vorzugsweise technische Kokos- oder Talgfettalkohole mit 12 bis 18, vorzugsweise mit 16 bis 18 Kohlenstoffatomen., sowie die einfach ungesättigten Fettalkohole, wie Oleylalkohol, Elaidylalkohol, Delta-9-cis-Hexadecenol, Delta-9-Octadecenol, trans-Delta-9-Octadecenol, cis-Delta-11-Octadecenol,trans-10,cis-12-Hexadecadien-1-ol, Octacosa-10,19-dien-1-ol und mehrfach ungesättigte Fettalkohole wie z.B. linoleyl alcohol (9Z, 12Z-octadecadien-1-ol), elaidolinoleyl alcohol (9E, 12E-octadecadien-1-ol), linolenyl alcohol (9Z, 12Z, 15Z-octadecatrien-1-ol), elaidolinolenylalcohol (9E, 12E, 15-E-octadecatrien-1-ol), wobei Mischungen von Kokos- oder Talgfettalkohole mit 16 bis 18 Kohlenstoffatomen besonderes bevorzugt sind.

Es hat sich als vorteilhaft erwiesen, wenn die erfindungsgemäßen Formulierungen zusätzlich 0,1 bis 10 Gew.-%, bevorzugt 0,25 bis 5 Gew.-%, insbesondere 0,5 bis 2,5 Gew.-%, mindestens eines Emulgators, enthalten, wobei sich die Gewichtsprozente auf die Gesamtformulierung beziehen.

In diesem Zusammenhang bevorzugte Emulgatoren sind ausgewählt aus der Gruppe der Fettalkoholalkoxylate, insbesondere der Fettalkoholethoxylate. Besonders bevorzugt enthaltene Fettalkoholethoxylate sind ausgewählt aus der Gruppe umfassend Polyoxyethylenether des Laurylalkohols, CAS-Nummer 9002-92-0, Macrogollaurylether, z. B. Polyoxyethylen (4) laurylether (Laureth-4, INCI), Polyoxyethylen (9) laurylether Laureth-9 (INCI), Polyoxyethylen (23) laurylether Laureth-23 (INCI) Polyoxyethylenether des Cetylalkohols, CAS-Nummer 9004-95-9, z.B. Polyoxyethylen (2) cetylether Ceteth-2 (INCI), Polyoxyethylen (10) cetylether Ceteth-10 (INCI) Polyoxyethylen (20) cetylether Ceteth-20 (INCI) Polyoxyethylenether des Cetylstearylalkohols, CAS-Nummer 68439-49-6, z.B. Polyoxyethylen (6) cetylstearylether Ceteareth-6 (INCI) Polyoxyethylen (20) cetylstearylether Ceteareth-20 (INCI) Polyoxyethylen (25) cetylstearylether Ceteareth-25 (INCI) Polyoxyethylenether des Stearylalkohols, CAS-Nummer 9005-00-9, z.B. Polyoxyethylen (2) stearylether Steareth-2 (INCI) Polyoxyethylen (10) stearylether Steareth-10 (INCI) Polyoxyethylen (20) stearylether Steareth-20 (INCI) Polyoxyethylenether des Oleylalkohols, CAS-Nummer 9004-98-2, z.B. Polyoxyethylen (2) oleylether Oleth-2 (INCI) Polyoxyethylen (10) oleylether Oleth-10 (INCI) Polyoxyethylen (20) oleylether Oleth-20 (INCI)
oder Polyoxyethylen (10) tridecylether (CAS-Nummer 24938-91-8) und Trideceth-10 (INCI).

Alternativ bevorzugte Emulgatoren sind ausgewählt aus der Gruppe der Polyolester, insbesondere der Glycerinester und Polyglycerinester, insbesondere der Polyglycerinester. Bevorzugt enthaltene (Poly)glycerinester sind dadurch gekennzeichnet, dass sie Partialester sind. Besonders bevorzugte Polyglycerinpartialester sind ausgewählt aus der Gruppe umfassend Polyglycerinpartialester wie in EP-B-0 835 862 beschrieben, die erhältlich sind durch Veresterung eines Polyglyceringemisches mit einem Veresterungsgrad des Polyglycerins zwischen 30 und 75 % und gesättigten oder ungesättigen, linearen oder verzweigten Fettsäuren mit 12 bis 22 C-Atomen und Dimerfettsäuren mit einer mittleren Funktionalität von 2 bis 2,4, Ester der Zitronensäure wie etwa der O/W-Emulgator Glycerylstearatcitrat, (2-Hydroxy-1,2,3-propantricarbonsäure-1,2,3-propantriolmonooctadecanoat, INCI Glyceryl Stearate Citrate, CAS 39175-72-9), der Citronensäureester des Glycerylstearats, u.a. unter der Bezeichnung AXOL C 62 im Handel erhältlich, Glycerylstearatcitrat wie in WO2006034992 und WO2008092676 beschrieben und Glyceryloleatcitrat wie in WO2004112731 beschrieben, ebenso einfache Polyglycerinester, wie beispielsweise Polygylcerin-3 Distearate, Polyglyceryl-10 Stearate, Polyglyceryl-6 Distearate, Mischester aus Polyglycerin und Methylglucose und Stearinsäure, wie beispielsweise Polyglyceryl-3 Methylglucose Distearate und (Poly-)glycerinpartialester mit einer oder mehrerer Carbonsäuren mit 10 bis 24 Kohlenstoffatomen und Resten einer polyfunktionalen Carbonsäure.

Prinzipiell können auch Sorbitan- oder Saccharoseester als Polyolester eingesetzt werden. Eine gängige Kombination ist beispielsweise Sorbitan Stearate & Sucrose Cocoate.

In einer weiteren Alternative bevorzugt enthaltene Emulgatoren sind ausgewählt aus der Gruppe der modifizierten Siloxane, beispielsweise solche, die neben aliphatischen Gruppen auf Basis von alpha-Olefinen auch Polyether tragen. Siloxanbasierte Emulgatoren für Öl-in-Wasser-Emulsionen müssen einen hydrophilen Charakter aufweisen, weshalb es sich in der Regel um reine Polyethersiloxane handelt. Besonder geeiognete Beispiele sind relativ hydrophobe Polyethersiloxane wie in EP 1125574 beschrieben, hochmolekulare Polyethersiloxane wie in EP2168564 beschrieben und organomodifizierte Siloxanblockcopolymere wie in WO2009138306 beschrieben. Bevorzugt enthaltene modifizierte Siloxane sind dadurch gekennzeichnet, dass sie einen HLB-Wert >8 aufweisen. Besonders bevorzugte modifizierte Siloxane sind ausgewählt aus der Gruppe umfassend Bis-PEG/PPG-16/16 Dimethicone, PEG/PPG-16/16 Dimethicone, Bis-PEG/PPG-20/5 PEG/PPG-20/5 Dimethicone und Methoxy PEG/PPG-25/4 Dimethicone.

Vorgenannte Emulgatoren ergeben im Zusammenhang mit der vorliegenden Erfindung besonders lagerstabile Formulierungen.

Erfindungsgemäße Formulierungen enthaltend eine Verbindung der allgemeinen Formel I) 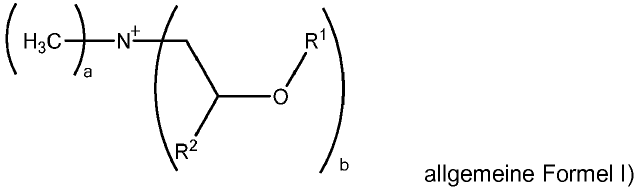
mit R¹ ein Acylrest der Isostearinsäure,
mit R² ein Alkylrest mit 1 bis 6 Kohlenstoffatomen, bevorzugt Methyl, Ethyl, Propyl, Isopropyl, besonders bevorzugt Methyl,
wobei a = 1 bis 3 und b = 1 bis 3, mit der Maßgabe, dass a + b =4, sind bevorzugt dadurch gekennzeichnet, dass sie als Emulgator solche ausgewählt aus der Gruppe ethoxylierte Emulgatoren, wie z.B. TEGINACID (Glyceryl Stearate, Ceteareth-20) oder TEGINACID C (Ceteareth-25), oder TEGO Acid S 40 P (PEG-40 Stearate) oder TEGO Alkanol IC 20 (lsoceteth-20) oder Laureth-23 oder Steareth-20 oder Steareth-21 oder TEGO Care 165 (Glyceryl Stearate, PEG-100 Stearate) oder TEGO Care 215 (Ceteareth-15, Glyceryl Stearate), organomodifizierte Siloxane, wie z.B. ABIL Care 85 (Bis-PEG/PPG-16/16 PEG/PPG-16/16 Dimethicone;Caprylic/Capric Triglyceride) oder ABIL Care XL 80 (Bis-PEG/PPG-20/5 PEG/PPG-20/5 Dimethicone; Methoxy PEG/PPG-25/4 Dimethicone; Caprylic/Capric Triglyceride, kationische Tenside, wie z.B. VARISOFT PATC (Palmamidopropyltrimonium Chloride) oder VARISOFT TA 100 (Distearyldimonium Chloride) oder VARISOFT BT 85 (Behentrimonium Chloride), zuckerbasierte Emulgatoren, wie z.B TEGO Care PS (Methyl Glucose Sesquistearate) oder TEGO Care CG 90 (Cetearyl Glucoside), glycerinbasierte Emulgatoren, wie z.B. AXOL C62 Pellets (Glyceryl Stearate Citrate) oder TEGO Care PL 4 (Polyglyceryl-4 Laurate), TEGO Care PSC 3 (Polyglyceryl-3 Stearate/Citrate), zucker- und glycerinbasierte Emulgatoren, wie z.B. TEGO Care 450 (Polyglyceryl-3 Methylglucose Distearate) oder TEGO Care PS (Methyl Glucose Sesquistearate) und Mischungen aus den genannten Gruppen, wie z.B. TEGO Care LTP (Sorbitan Laurate, Polyglyceryl-4 Laurate, Dilauryl Citrate) enthalten.

Die erfindungsgemäßen Formulierungen können z.B. mindestens eine weitere, zusätzliche Komponente enthalten, ausgewählt aus der Gruppe der
Emollients,
Co-Emulgatoren,
Verdicker/Viskositätsregler/Stabilisatoren ,
Antioxidantien,
Hydrotrope (oder Polyole),
Fest- und Füllstoffe,
Perlglanzadditive,
Deodorant- und Antitranspirantwirkstoffe,
Insektrepellentien,
Selbstbräuner,
Konservierungsstoffe,
Konditioniermittel,
Parfüme,
Farbstoffe,
kosmetische Wirkstoffe,
Pflegeadditive,
Überfettungsmittel,
Lösungsmittel.

Substanzen, die als beispielhafte Vertreter der einzelnen Gruppen eingesetzt werden können, sind dem Fachmann bekannt und können beispielsweise der deutschen Anmeldung DE 102008001788.4 entnommen werden. Diese Patentanmeldung wird hiermit als Referenz eingeführt und gilt somit als Teil der Offenbarung.

Bezüglich weiterer fakultativer Komponenten sowie der eingesetzten Mengen dieser Komponenten wird ausdrücklich auf die dem Fachmann bekannten einschlägigen Handbücher, z. B. K. Schrader, "Grundlagen und Rezepturen der Kosemtika", 2. Auflage, Seite 329 bis 341, Hüthig Buch Verlag Heidelberg, verwiesen.

Die Mengen der jeweiligen Zusätze richten sich nach der beabsichtigten Verwendung. Typische Rahmenrezepturen für die jeweiligen Anwendungen sind bekannter Stand der Technik und sind beispielsweise in den Broschüren der Hersteller der jeweiligen Grund- und Wirkstoffe enthalten. Diese bestehenden Formulierungen können in der Regel unverändert übernommen werden. Im Bedarfsfall können zur Anpassung und Optimierung die gewünschten Modifizierungen aber durch einfache Versuche komplikationslos vorgenommen werden.

Die erfindungsgemäße Formulierung kann zusätzlich mindestens eine Verbindung der allgemeinen Formel II) 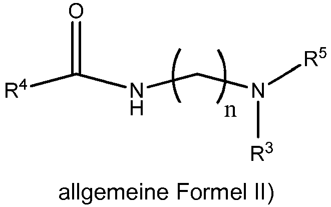 mit
R⁴-CO ausgewählt aus einem aliphatischen, linearen oder verzweigten Acylrest mit 6 bis 22 Kohlenstoffatomen enthaltend 0, 1, 2 oder 3 Doppelbindungen, der in der Regel aus einer natürlich vorkommenden oder synthetisch hergestellten Fettsäure stammt,
R⁵ und R³ ausgewählt aus gleiche oder unterschiedliche Alkylreste, die gegebenenfalls funktionelle Gruppen wie Hydroxygruppen, Estergruppen, Amine, Amide oder sonstige polare Substituenten tragen können, wobei unsubstituierte Kohlenwasserstoffreste, die allenfalls eine oder mehrere Verzweigungen aufweisen, bevorzugt sind, insbesondere bevorzugt sind Kohlenwasserstoffreste mit 1 bis 6 C-Atomen, wobei Ethyl und Methylreste erfindungsgemäß ganz besonders bevorzugt sind,
n = eine ganze Zahl ausgewählt aus von 1 bis 10, bevorzugt von 2 bis 7, insbesondere von 2 bis 4,
   enthalten.

Die Verbindungen der allgemeinen Formel II) werden üblicherweise durch Amidierung natürlicher oder synthetischer Fettsäuren und Fettsäureschnitte mit Dialkylaminoaminen hergestellt. Typische Beispiele für solche Fettsäuren sind Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z. B. bei der Druckspaltung von natürlichen Fetten und Ölen, bei der Reduktion von Aldehyden aus der Roelenschen Oxosynthese oder der Dimerisierung von ungesättigten Fettsäuren anfallen.

Besonders bevorzugt sind üblicherweise die Fettsäureschnitte, welche aus Cocosöl oder Palmöl erhältlich sind, insbesondere bevorzugt ist in der Regel der Einsatz von Stearinsäure.

Die erfindungsgemäße Formulierung kann je nach Einsatzzweck die Verbindung der allgemeinen Formel I), II) und gegebenenfalls Ia) in unterschiedlichen Verhältnissen enthalten.

Bevorzugt sind die Verbindungen der allgemeinen Formel I) und gegebenenfalls Ia) (dann in Summe) zu den Verbindungen der allgemeinen Formel II) in einem Gewichtsverhältnis von 20 zu 1 bis 1 zu 20, insbesondere von 10 zu 1 bis 1 zu 10 in der erfindungsgemäßen Formulierung enthalten. Je nach gewünschtem Effekt kann das Gewichtsverhältnis auch von 5 zu 1 bis 1 zu 5 oder von 3 zu 1 bis 1 zu 3 variiert werden.

Erfindungsgemäß bevorzugte Formulierungen, insbesondere solche zur Behandlung keratinischer Fasern, insbesondere von menschlichem Haar, enthalten gegebenenfalls in Summe 0,1 bis 7 Gew.-%, bevorzugt 0,2 bis 5 Gew.-% und insbesondere bevorzugt 0,3 bis 3 Gew.-%, Verbindungen der allgemeinen Formel I) und gegebenenfalls Ia) und gegebenenfalls zusätzlich
0,1 bis 7 Gew.-%, bevorzugt 0,2 bis 5 Gew.-% und insbesondere bevorzugt 0,3 bis 3 Gew.-%, Verbindungen der allgemeinen Formel II),
wobei sich die Gew.-% auf die Gesamtformulierung beziehen.

Besonders gute Ergebnisse lassen sich bei den vordefinierten Konzentrationen von 0,2 bis 2 Gew.-% erzielen. Die Anwendung der erfindungsgemäßen Formulierungen an keratinischen Fasern, insbesondere an menschlichen Haaren ist jedoch nicht auf den Einsatz der Wirkstoffe in geringer Konzentration limitiert. Es können ebenso konzentrierte erfindungsgemäße Formulierungen zur Anwendung kommen, in denen die vordefinierten Konzentrationen 2 bis 20 Gew.-% oder 3 bis 14 Gew.-%, insbesondere 5 bis 12 Gew.-%, betragen.

Es ist erfindungsgemäß bevorzugt, wenn die Formulierung einen pH-Wert von 3,0 bis 5,5 aufweist, bevorzugt 3,5 bis 5,0.

Die Ladung der in der erfindungsgemäßen Formulierung enthaltenen Verbindung 1 muss durch entsprechende Anionen kompensiert werden; dies findet durch in der erfindungsgemäßen Formulierung enthaltene Gegenanionen statt.

Als solche Gegenanionen sind beispielsweise die Halogenide, Pseudohalogenide, Anionen von Mineralsäuren, Sulfate, Sulfite, Hydrogensulfite, Sulfonat, Alkyl- und Arylsulfonate, Phosphat, Hydrogenphosphate, Phosphite, Hydrogenphosphite, Phosphonite, Carboxylate, Borate, Carbonate, Sulfide, Hydrogensulfide, Lactat, Glycolat, Formiat, Acetat und Propionat geeignet.

Diese Anionen sind bevorzugt ausgewählt aus solchen, die für kosmetische Anwendung geeignet und somit beispielsweise nicht toxisch sind. Besonders bevorzugt ist mindestens ein Gegenanion zu der Verbindung der allgemeinen Formel I) ausgewählt aus der Gruppe umfassend Chlorid, Bromid, lodid, Alkylsulfat, z.B. Methylsulfat, Ethylsulfat, Alkylsulfonat, z.B. Methylsulfonat, Triflat, Tosylat, Phosphat, Sulfat, Hydrogensulfat, Lactat, Glycolat, Acetat und Citrat, bevorzugt Chlorid und Methylsulfat, enthalten.

Die Verbindung der allgemeinen Formel I) 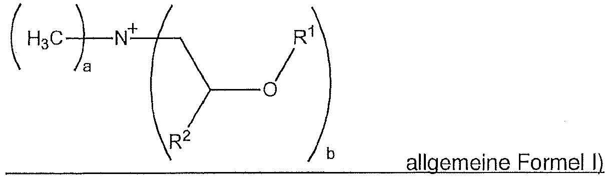
mit R¹ ein Acylrest der Isostearinsäure,
mit R² ein Alkylrest mit 1 bis 6 Kohlenstoffatomen,
wobei a = 1 bis 3 und b = 1 bis 3,
mit der Maßgabe, dass a + b =4.
sowie die erfindungsgemäßen Formulierungen lassen sich erfindungsgemäß für die Behandlung keratinischer Fasern, insbesondere für die Haarbehandlung, verwenden. allgemeinen

Die erfindungsgemäße Verwendung führt zur Verbesserung der Konditionierung, des Glanzes, der Flexibilität, der Spannkraft, und/oder der Kämmbarkeit sowie einer Reduzierung der Bruchwahrscheinlichkeit der behandelten Faser und außerdem reduziert sie die antistatischen Kräfte zwischen den Fasern.

Die erfindungsgemäße Verwendung führt zum Schutz der Fasern vor Hitze.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.

Folgende Abbildungen sind Bestandteil der Beispiele:
Abbildung 1: Kämmkraftmessungen Vergleich Formulierung 1b mit V3b
Abbildung 2: Kämmkraftmessungen Vergleich Formulierung V6b mit V3b
Abbildung 3: Kämmkraftmessungen Vergleich Formulierung 5b mit V3b und V4b
Abbildung 4: Kämmkraftmessungen Vergleich Formulierung 7b mit V3b und V4b

### Beispiele:

### Beispiel 1: Herstellung von "1-Propanaminium, 2-hydroxy-N-(2-hydroxypropyl)-N,N-dimethyl-, diester mit Pflanzenmischölfettsäure, Methylsulfat" (erfindungsgemäß)

1120 g (4 mol) Pflanzenmischölfettsäure werden mit 302 g (2,05 mol) Methyldiisopropanolamin gemischt unter Rühren auf 180 °C aufgeheizt. Reaktionswasser wird kontinuierlich abdestilliert. Nachdem die Hauptmenge Reaktionswassser bei Normaldruck destilliert ist, wird Vakuum angelegt und die Säurezahl der Reaktionsmischung auf < 7 mg KOH/g abreagiert. Das entstandene Esteramin wird auf 60 °C abgekühlt und portionsweise mit 240 g (1,90 mol) Dimethylsulfat versetzt, so dass die Reaktionstemperatur 100 °C nicht übersteigt. Nach dem Abkühlen auf Raumtemperatur wird die Gesamt Amin Zahl (GAZ) und der Aktivgehalt des Fertigprodukts analysiert.

GAZ = 5,0 mg KOH/g; Aktivgehalt 1,27meq/g (Kationischer Aktivgehalt nach Epton).

### Beispiel 2: Herstellung von"1-Propanaminium, 2-hydroxy-N-(2-hydroxypropyl)-N,N-dimethyl-, diester mit Palmfettsäure, Methylsulfat" (nicht erfindungsgemäß)

1020 g (4 mol) Palmitinsäure (technische Qualität, ca. 98 % rein ) werden mit 302 g (2,05 mol) Methyldiisopropanolamin versetzt und wie unter Beispiel 1 beschrieben verestert. Das Esteramin hatte eine Säurezahl von 5,6 mg KOH/g. Diese Mischung wurde wie unter Beispiel 1 beschrieben mit 240 g (1,90 mol) Dimethylsulfat alkyliert. Die GAZ des Fertigprodukts wurde mit 4,8 mg KOH/g bestimmt, der Aktivgehalt war 1,33 meq/g.

### Beispiel 3: Herstellung von "1-Propanaminium, 2-hydroxy-N-(2-hydroxypropyl)-N,N-dimethyl-, diester mit Tallölfettsäure, Methylsulfat" (erfindungsgemäß)

814 g (2,84 mol) Tallölfettsäure werden mit 214 g (1,46 mol) Methyldiisopropanolamin gemischt und wie unter Beispiel 1 beschrieben verestert. Das Esteramin hatte eine Säurezahl von 3,2 mg KOH/g. Diese Mischung wurde wie in Beispiel 1 beschrieben mit 168 g (1,33 mol) Dimethylsufat alkyliert. Die GAZ des Fertigprodukts wurde mit 7,3 mg KOH/g bestimmt, der Aktivgehalt war 1,24 meq/g.

### Beispiel 4: Herstellung von "1-Propanaminium, 2-hydroxy-N-(2-hydroxypropyl)-N, N-dimethyl-, diester mit Erucasäure, Methylsulfat" (nicht erfindungsgemäß)

392 g (1,15mol) Erucasäure werden mit 99,3 g (0,68mol) Methyldiisopropanolamin gemischt und wie unter Beispiel 1 beschrieben verestert. Das Esteramin hatte eine Säurezahl von 2,2 mg KOH/g. Diese Mischung wurde wie in Beispiel 1 beschrieben mit 72,2 g (0,57mol) Dimethylsufat alkyliert. Die GAZ des Fertigprodukts wurde mit 4,6 mg KOH/g bestimmt, der Aktivgehalt war 1,45meq/g.

### Beispiel 5: Herstellung von "1-Propanaminium, 2-hydroxy-N-(2-hydroxypropyl)-N,N-dimethyl-, diester mit Isostearinsäure, Methylsulfat" (erfindungsgemäß)

1017 g (3,5 mol) Isostearinsäure (technische Qualität) werden mit 262 g (1,79 mol) Methyldiisopropanolamin versetzt und wie unter Beispiel 1 beschrieben verestert. Das Esteramin hatte eine Säurezahl von 3,4 mg KOH/g. Diese Mischung wurde wie unter Beispiel 1 beschrieben mit 209 g (1,66 mol) Dimethylsulfat alkyliert.

Die GAZ des Fertigprodukts wurde mit 5,1 mg KOH/g bestimmt, der Aktivgehalt war 1,26 meq/g.

### Beispiel 6: Anwendungstechnik von Haarbehandlungsmitteln unter Verwendung von Beispiel 1 und Beispiel 2 sowie kommerziellen Marktprodukten.

Für die anwendungstechnische Beurteilung wurden Haartressen verwendet, die durch eine Bleichbehandlung standardisiert vorgeschädigt wurden. Dazu werden friseurübliche Produkte eingesetzt. Die Schädigung der Haartressen wird im Detail in der DE10327871 beschrieben.

Für die anwendungstechnische Beurteilung wurde die erfindungsgemäße Verbindung aus Beispiel 1 in einer einfachen kosmetischen Formulierung eingesetzt.

Als Referenzverbindungen wurden das kommerziell erhältliche Alkylquat (INCI) Behentrimonium Chloride (VARISOFT^{®} BT 85 Pellets, Evonik Industries) und das kommerziell erhältliche Esterquat (INCI) DistearoylethylDimonium Chloride (VARISOFT^{®} EQ 65 Pellets, Evonik Industries), sowie die Verbindung Dimethyl-di-(palmitoyl-oxyisopropyl-)ammoniumchlorid verwendet.

Die Anwendungseigenschaften beim Einsatz in Haarspülungen wurden in den folgenden Rezepturen überprüft (Tab. 1):

**Tab. 1: Haarspung-ormuerung zur Austestung der haarkonditionierenden Eigenschaften**

| Formulierungsbeispiele | C0a | 1a | V2a | V3a | V4a | 5a | V6a | 7a |
|---|---|---|---|---|---|---|---|---|
| TEGINACID^{®} C, Evonik Industries (INCI: Ceteareth-25) | 0,5% | 0,5% | 0,5% | 0,5% | 0,5% | 0,5% | 0,5% | 0,5% |
| TEGO^{®}Alkanol 18, Evonik Industries (INCI: Stearylalkohol) | 4% | 4% | 4% | 4% | 4% | 4% | 4% | 4% |
| Beispiel 1 (100%) | | 1,5% | | | | | | |
| Beispiel 2 (80% in Isopropanol) | | | 1,9% | | | | | |
| Beispiel 3 | | | | | | 1,5% | | |
| Beispiel 4 | | | | | | | 1,5% | |
| Beispiel 5 | | | | | | | | 1,5% |
| VARISOFT^{®} EQ 65 Pellets (65%ig in C16 Fettalkohol), Evonik Industries (INCI: DistearoylethylDimonium Chloride, CetearylAlkohol) | | | | 2,3% | | | | |
| VARISOFT^{®} BT 85, (85%ig in Isopropanol), Evonik Industries (INCI: Behentrimonium Chloride) | | | | | 1,76% | | | |
| Wasser, demineralisiert | ad 100,0 | | | | | | | |
| Zitronensäure | ad pH 4,0 | | | | | | | |

Die Zusammensetzung der Testformulierungen ist zur Vermeidung der Beeinflussung der Testergebnisse durch (normalerweise vorhandene)Formulierungsbestandteile bewusst einfach gewählt. Erfindungsgemäße Formulierungen können neben den genannten Inhaltsstoffen und/oder anstatt der genannten Inhaltsstoffe noch weitere Inhaltsstoffe enthalten. Insbesondere die Kombination mit weiteren Inhaltsstoffen kann bei den beschriebenen Effekten zu einer synergistischen Verbesserung führen.

Die Vorbehandlung der Haare erfolgt durch eine Shampooformulierung (Tab. 2), welche keine Konditioniermittel enthält.

**Tab. 2: Shampooformulierung für die Vorbehandlung der Haartressen.**

| | |
|---|---|
| Texapon NSO^{®}, 28%-ig, Cognis (INCI: SodiumLaureth Sulfate) | 42,9% |
| NaCl | 3% |
| Wasser, demineralisiert | ad 100,0 |

Standardisierte Behandlung von vorgeschädigten Haarsträhnen mit konditionierenden Proben:
Die wie oben beschrieben vorgeschädigten Haarsträhnen werden mit der Shampooformulierung aus Tab. 2 gewaschen.

Hierbei werden die Haarsträhnen unter fließendem, warmem Wasser benetzt. Das überschüssige Wasser wird leicht von Hand ausgedrückt, dann wird das Shampoo aufgebracht und 1 min lang sanft ins Haar eingearbeitet (0.5 ml/2 g Haarsträhne). Die Haartresse wird für 30 s unter fließendwarmem Wasser ausgespült. Diese Prozedur wird ein weiteres mal wiederholt, nur dass abschließend 1 min lang ausgespült wird. Anschließend werden direkt nach dem Waschen die Haarsträhnen mit den Haarspülungs-Formulierungen aus Tab. 1 konditioniert.

Hierbei wird die Spülung aufgebracht und sanft ins Haar eingearbeitet (0.5 ml/2 g Haarsträhne). Nach einer Verweilzeit von 1 min wird das Haar für a) 1 min oder für b) 3 min gespült.

Vor der sensorischen Beurteilung wird das Haar an der Luft bei 50% Luftfeuchtigkeit und 25°C für mindestens 12 h getrocknet.

Beurteilungskriterien:
Die sensorischen Bewertungen erfolgen nach Noten, die auf einer Skala von 1 bis 5 vergeben werden, wobei 1 die schlechteste und 5 die beste Bewertung ist. Die einzelnen Testkriterien erhalten jeweils eine eigene Bewertung.

Die Testkriterien sind:
Nasskämmbarkeit, Nassgriff, Trockenkämmbarkeit, Trockengriff, Aussehen/Glanz.

In den folgenden Tabellen werden die Ergebnisse der sensorischen Beurteilung der wie oben beschrieben durchgeführten Behandlung der Haarsträhnen bei a) 1 min Ausspülzeit und bei b) 3 min Ausspülzeit mit der erfindungsgemäßen Formulierung 1a den Vergleichsformulierungen V2a, V3a und V4a und der Kontrollformulierung C0a (Control ohne Testsubstanz) verglichen und bei aa) 1 min Ausspülzeit und bei bb) 3 min Ausspülzeit mit der erfindungsgemäßen Formulierung 7a, den Vergleichsformulierungen V2a, V3a und V4a und der Kontrollformulierung C0a (Control ohne Testsubstanz) verglichen

### a) 1 min Ausspülzeit

**Tab. 3a: Ergebnisse der Konditionierung von Haar bei 1 min Ausspülzeit**

| | Nasskämmbarkeit | Nassgriff | Trockenkämmbarkeit | Trockengriff |
|---|---|---|---|---|
| Erfindungsgemäße Formulierung 1a | 5 | 5 | 5 | 5 |
| Vergleichsformulierung 2a | 3,5 | 3,5 | 3,5 | 3,5 |
| Vergleichsformulierung 3a | 3,5 | 3,5 | 4 | 3,5 |
| Vergleichsformulierung 4a | 4,5 | 4,5 | 4,5 | 4,5 |
| Kontrolle C0a | 2 | 2 | 3 | 2,5 |

### b) 3 min Ausspülzeit

**Tab. 3b: Ergebnisse der Konditionierung von Haar bei 3 min Ausspülzeit**

| | Nasskämmbarkeit | Nassgriff | Trockenkämmbarkeit | Trockengriff |
|---|---|---|---|---|
| Erfindungsgemäße Formulierung 1a | 5 | 5 | 5 | 5 |
| Vergleichsformulierung 2a | 3,0 | 3,0 | 3,5 | 3,5 |
| Vergleichsformulierung 3a | 3,5 | 3,5 | 4 | 3 |
| Vergleichsformulierung 4a | 4 | 4 | 4 | 4 |
| Kontrolle C0a | 2 | 2 | 3 | 2,5 |

Die Ergebnisse in Tabelle 3a zeigen, dass die erfindungsgemäße Formulierung 1a bei 1 min Ausspülzeit sehr gute konditionierende Eigenschaften aufweist, die den Vergleichsformulierungen V2a, V3a und V4a signifikant überlegen sind.

Die Ergebnisse in Tabelle 3b zeigen, dass die erfindungsgemäße Formulierung 1a bei 3 min Ausspülzeit noch deutlicher als nach 1 min verbesserte konditionierende Eigenschaften als die Vergleichsformulierungen V2a, V3a und V4a aufweist. Die Vergleichsformulierung V4a enthält als konditionierende Verbindung VARISOFT^{®}BT 85 (85%ig in Isopropanol, Evonik Industries, INCI: Behentrimonium Chloride), ein Alkylquat, das für seine sehr guten konditionierenden Eigenschaften auch bei langen Ausspülzeiten bekannt ist. Die Vergleichsformulierung V3a enthält als konditionierende Verbindung VARISOFT^{®} EQ 65Pellets (65%ig in C 16 Fettalkohol, Evonik Industries, INCI: DistearoylethylDimonium Chloride, CetearylAlcohol), ein Esterquat, das sehr gute konditionierenden Eigenschaften bei 1 min Ausspülzeit aufweist (siehe Tab. 3a), bei 3 min Ausspülzeit jedoch eine deutlich schlechtere Konditionierung zeigt.

### aa) 1 min Ausspülzeit

**Tab. 3c: Ergebnisse der Konditionierung von Haar bei 1 min Ausspülzeit**

| | Nasskämmbarkeit | Nassgriff | Trockenkämmbarkeit | Trockengriff |
|---|---|---|---|---|
| Erfindungsgemäße Formulierung 7a | 5 | 5 | 5 | 5 |
| Vergleichsformulierung 2a | 3,5 | 3,5 | 3,5 | 3,5 |
| Vergleichsformulierung 3a | 3,5 | 3,5 | 4 | 4 |
| Vergleichsformulierung 4a | 4,5 | 4,5 | 4,5 | 4,5 |
| Kontrolle C0a | 2 | 2 | 3 | 2,5 |

### bb) 3 min Ausspülzeit

**Tab. 3d: Ergebnisse der Konditionierung von Haar bei 3 min Ausspülzeit**

| | Nasskämmbarkeit | Nassgriff | Trockenkämmbarkeit | Trockengriff |
|---|---|---|---|---|
| Erfindungsgemäße Formulierung 7a | 5 | 5 | 5 | 5 |
| Vergleichsformulierung 2a | 3,0 | 3,0 | 3,5 | 3,5 |
| Vergleichsformulierung 3a | 3,5 | 3,0 | 3,5 | 3,5 |
| Vergleichsformulierung 4a | 4 | 4 | 4 | 4 |
| Kontrolle C0a | 2 | 2 | 3 | 2,5 |

Die Ergebnisse in Tabelle 3c zeigen, dass die erfindungsgemäße Formulierung 7a bei 1 min Ausspülzeit sehr gute konditionierende Eigenschaften aufweist, die den Vergleichsformulierungen V2a, V3a und V4a signifikant überlegen sind.

Die Ergebnisse in Tabelle 3d zeigen, dass die erfindungsgemäße Formulierung 7a bei 3 min Ausspülzeit noch deutlicher als nach 1 min verbesserte konditionierende Eigenschaften als die Vergleichsformulierungen V2a, V3a und V4a aufweist. Die Vergleichsformulierung V4a enthält als konditionierende Verbindung VARISOFT^{®}BT 85 (85%ig in Isopropanol, Evonik Industries, INCI: Behentrimonium Chloride), ein Alkylquat, das für seine sehr guten konditionierenden Eigenschaften auch bei langen Ausspülzeiten bekannt ist. Die Vergleichsformulierung V3a enthält als konditionierende Verbindung VARISOFT^{®} EQ 65Pellets (65%ig in C 16 Fettalkohol, Evonik Industries, INCI: DistearoylethylDimonium Chloride, CetearylAlcohol), ein Esterquat, das sehr gute konditionierenden Eigenschaften bei 1 min Ausspülzeit aufweist (siehe Tab. 3a), bei 3 min Ausspülzeit jedoch eine deutlich schlechtere Konditionierung zeigt.

### Beispiel 7: Einfluss der erfindungsgemäßen Verbindungen auf Kämmkräfte von Haaren

Versuchsbedingungen:
Messgerät: Diastron MTT 175
Messstrecke: 20 cm
Kämmgeschwindigkeit: 2000 mm/min
Verwendete Haarsträhnen: Länge = 23 cm; Breite = 1,5cm; Gewicht = 2 g Messbedingungen: T = 22°C.

Die Haarsträhnen werden mit einer Restfeuchte von 60%, bestimmt durch Gewichtsbestimmung, gemessen.

Für die Versuche wird europäisches, ungeschädigtes, dunkelbraunes Haar eingesetzt. Zur Durchführung der Kämmkraftmessungen wird dieses Haar im Labor nach Standardbedingungen durch Dauerwelle geschädigt:
1.) 4 g Dauerwell-Lösung/g Haar, 15 min. einwirken lassen, 2 min. ausspülen unter fließendem Leitungswasser (T = 35°C). (Dauerwell-Lösung: Universal-Dauerwelle, Basler) 2.) 4 g Fixierer (1 Teil Fixierlösung. + 3 Teile Wasser)/g Haar, 10 min. einwirken lassen, 2 min. ausspülen. (Fixierlösung: Schaumfixierung Konzentrat, Basler) Durchführung der Kämmkraftmessung vor der Behandlung mit der Testformulierung:
Die vorgeschädigten Haarsträhnen werden über Nacht klimatisiert.
3.) Die Haarsträhne wird 1, min in einer Pufferlösung (Na-Citrat, pH = 6) eingetaucht.
4.) Die Haarsträhne wird so lange von Hand vorgekämmt, bis keine Änderung des Kämmwiderstandes festzustellen ist. 5.) Die Haarsträhne wird am Messgerät befestigt und die erste Kämmkraftmessung wird durchgeführt. Die Messung wird insgesamt 10 mal wiederholt.

### Behandlung der Strähnen:

Pro Haarsträhne werden 0,5 g der jeweiligen Testformulierung verwendet (2 g Haar/0,5 g Lösung). Die Formulierung wird 30 sec. in das Haareinmassiert und dann für 5 min. liegen gelassen, anschließend 1 min. bzw. 3 min. unter fließendem Leitungswasser abgespült.

Durchführung der Kämmkraftmessung nach der Behandlung mit der Testformulierung: Die Punkte 3-5 werden wiederholt.

Anschließend wird die Kämmarbeit (%) vor und nach der Behandlung mit der Testformulierung berechnet.

Verwendete Testformulierungen:
Die Kämmkräfte beim Einsatz in Haarspülungen wurden in den folgenden Rezepturen überprüft (Tab. 4):

**Tab. 4: Haarspülung-Formulierungen zur Austestung der haarkonditionierenden Eigenschaften**

| Formulierungsbeispiele | C0b | 1b | V2b | V3b | V4b | 5b | V6b | 7b |
|---|---|---|---|---|---|---|---|---|
| TEGINACID C, Evonik Industries (INCI: Ceteareth-25) | 0,5 % | 0,5 % | 0,5% | 0,5% | 0,5% | 0,5 % | 0,5 % | 0,5 % |
| TEGO Alkanol 18, Evonik Industries (INCI: Stearylalkohol) | 4% | 4% | 4% | 4% | 4% | 4% | 4% | 4% |
| Beispiel 1 (100%) | | 1% | | | | | | |
| Beispiel 2 (80% ig in Isopropanol) | | | 1,25 % | | | | | |
| Beispiel 3 | | | | | | 1% | | |
| Beispiel 4 | | | | | | | 1% | |
| Beispiel 5 | | | | | | | | 1% |
| Varisoft EQ 65 Pellets (65%ig in C16 Fettalkohol), Evonik Industries (INCI: Distearoylethyl Dimoniu m Chloride, CetearylAlkohol) | | | | 1,53 % | | | | |
| VARISOFT BT 85, (85%ig in Isopropanol), Evonik Industries (INCI: Behentrimonium Chloride) | | | | | 1,18 % | | | |
| Wasser, demineralisiert | ad 100,0 | | | | | | | |
| Zitronensäure | ad pH 4,0 | | | | | | | |

In der folgenden Abbildung werden die Ergebnisse der Kämmkraftmessungen der wie oben beschrieben durchgeführten Experimente bei a) 1 min Ausspülzeit und bei b) 3 min Ausspülzeit mit der erfindungsgemäßen Formulierung Ib, den Vergleichsformulierungen V3b und V4b und der Kontrollformulierung C0b (Control ohne Testsubstanz) verglichen.

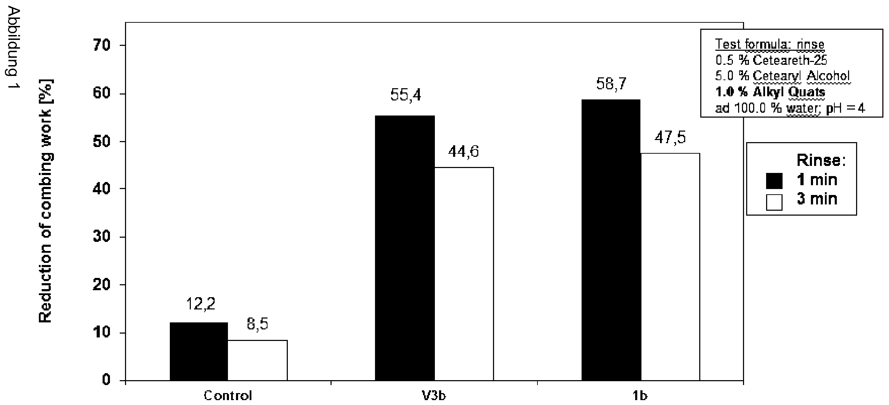

Die Ergebnisse in Abbildung 1 zeigen, dass die erfindungsgemäße Formulierung Ib bei 1 min Ausspülzeit eine ausgeprägtere Reduktion der Kämmkräfte als die Vergleichsformulierung V3b aufweist. Die Ergebnisse in Abbildung 1 zeigen weiterhin, dass die erfindungsgemäße Formulierung Ib bei 3 min Ausspülzeit eine ausgeprägtere Reduktion der Kämmkräfte als die Vergleichsformulierung V3b aufweist. Die Vergleichsformulierung V3b enthält als konditionierende Verbindung VARISOFT^{®} EQ 65 Pellets (65%ig in C 16 Fettalkohol, Evonik Industries, INCI: DistearoylethylDimonium Chloride, CetearylAlcohol), ein Esterquat.

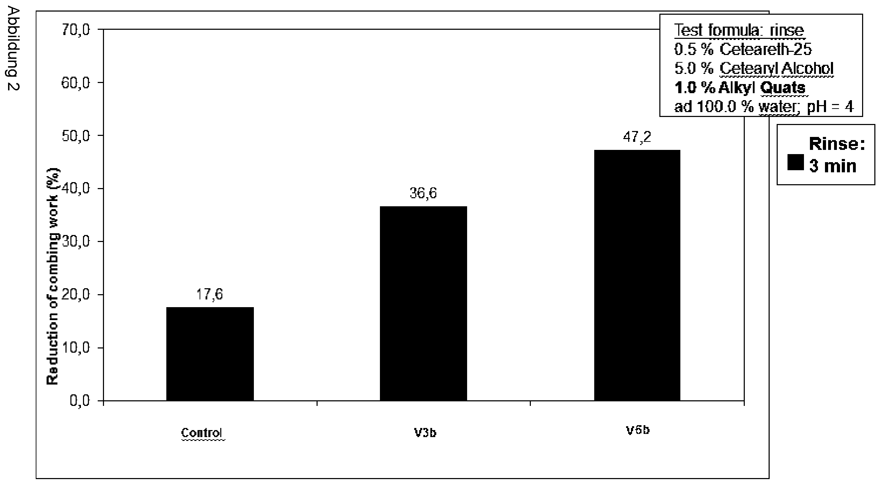

Die Ergebnisse in Abbildung 2 zeigen, dass die nicht erfindungsgemäße Formulierung V6b bei 3 min Ausspülzeit eine ausgeprägtere Reduktion der Kämmkräfte als die Vergleichsformulierung V3b aufweist.

Die Vergleichsformulierung V3b enthält als konditionierende Verbindung VARISOFT^{®} EQ 65 Pellets (65%ig in C 16 Fettalkohol, Evonik Industries, INCI: DistearoylethylDimonium Chloride, CetearylAlcohol), ein Esterquat.

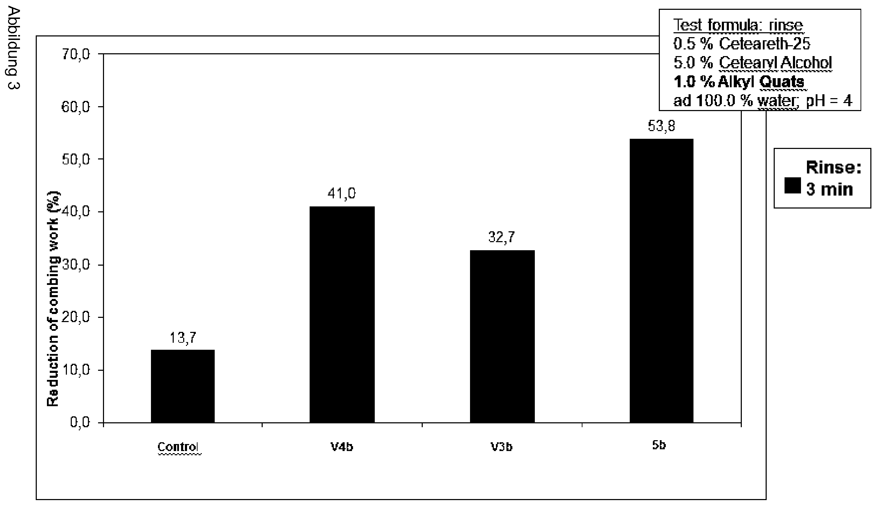

Die Ergebnisse in Abbildung 3 zeigen, dass die erfindungsgemäße Formulierung 5b bei 3 min Ausspülzeit eine ausgeprägtere Reduktion der Kämmkräfte als die Vergleichsformulierungen V3b und V4b aufweist.

Die Vergleichsformulierung V3b enthält als konditionierende Verbindung VARISOFT^{®} EQ 65 Pellets (65%ig in C 16 Fettalkohol, Evonik Industries, INCI: DistearoylethylDimonium Chloride, CetearylAlcohol), ein Esterquat. Die Vergleichsformulierung V4b enthält als konditionierende Verbindung VARISOFT^{®} BT 85 (85%ig in Isopropanol, Evonik Industries, INCI: Behentrimonium Chloride), ein Alkylquat, das für seine starke Reduktion der Kämmkräfte auch bei langen Ausspülzeiten bekannt ist.

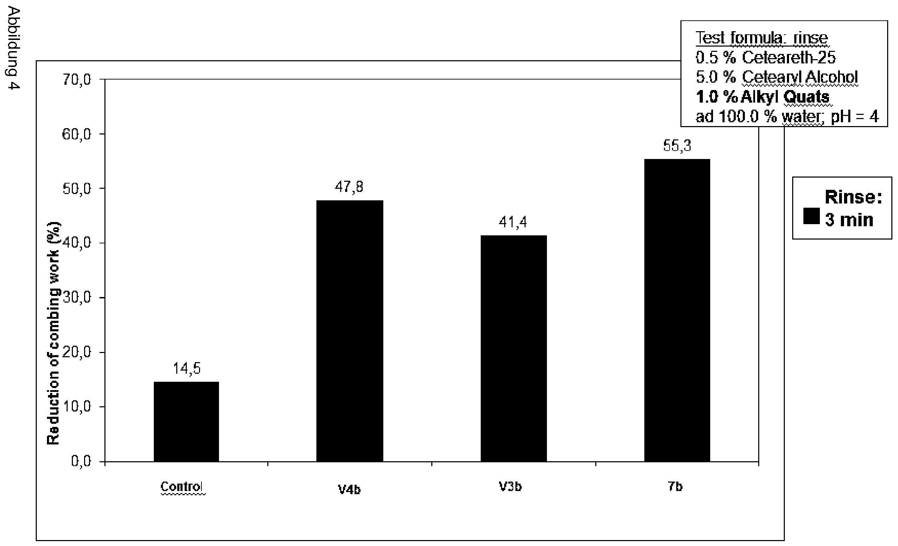

Die Ergebnisse in Abbildung 4 zeigen , dass die erfindungsgemäße Formulierung 7b bei 3 min Ausspülzeit eine ausgeprägtere Reduktion der Kämmkräfte als die Vergleichsformulierungen V3b und V4b aufweist.

Die Vergleichsformulierung V3b enthält als konditionierende Verbindung VARISOFT^{®} EQ 65 Pellets (65%ig in C 16 Fettalkohol, Evonik Industries, INCI: DistearoylethylDimonium Chloride, CetearylAlcohol), ein Esterquat. Die Vergleichsformulierung V4b enthält als konditionierende Verbindung VARISOFT^{®} BT 85 (85%ig in Isopropanol, Evonik Industries, INCI: Behentrimonium Chloride), ein Alkylquat, das für seine starke Reduktion der Kämmkräfte auch bei langen Ausspülzeiten bekannt ist.

### Beispiel 8: Antistatische Ausrüstung von keratinischen Fasern

Zur Austestung des antistatischen Verhaltens wurde die Schattensilhouette-Methode angewendet.

Die oben beschriebenen, vorbehandelten Haartressen, ein Plastikkamm, ein Punktstrahler und ein mit konzentrischen Halbkreisen gekennzeichnetes Projektionsfeld werden eingesetzt

Die Versuche wurden unter standardisierten klimatischen Bedingungen durchgeführt. Die Haartresse wird im Abstand von 15 cm vom Projektionsfeld aufgehängt. Der Punktstrahler wird in einer Entfernung von 145 cm von der Haartresse aufgestellt, so dass ein Schatten auf das Projektionsfeld fällt.

Nun wird die Haartresse mit dem Kamm fünfmal hintereinander gekämmt. Die elektrostatische Aufladung wird über die Schattensilhouette gemessen, indem die beiden äußeren Punkte des Schattens markiert werden und deren Abstand ermittelt wird. Je kleiner die Schattenfläche, umso effektiver ist die antistatische Wirkung.

Ergebnis:

| Formulierung | Abstand |
|---|---|
| C0a | 15 cm |
| 1a | 5 cm |

### Beispielformulierungen

**Formulierungsbeispiel 1) PearlizedShampoo**

| | |
|---|---|
| TEXAPON^{Ⓡ} NSO, Cognis, 28%-ig (INCI: SodiumLaureth Sulfate) | 32,25% |
| Erfindungsgemäßes Beispiel 1 | 0,25% |
| Perfume | 0,25% |
| Water | 55,25% |
| TEGO^{Ⓡ}Betain F 50, Evonik Industries AG, 38%-ig (INCI: CocamidopropylBetaine) | 8,00% |
| TEGO^{Ⓡ} Pearl N 300 Evonik Industries AG (INCI: Glycol | 2,00% |
| Distearate; Laureth-4; CocamidopropylBetaine) | |
| ANTIL^{Ⓡ} 171 Evonik Industries AG (INCI: PEG-18 Glyceryl Oleate/Cocoate) | 1,50% |
| NaCl | 0,50% |
| Preservative | q.s. |

**Formulierungsbeispiel 2) Rinse-Off Conditioner**

| | |
|---|---|
| Water | 92,0% |
| TEGINACID^{®} C, Evonik Industries AG (INCI: Ceteareth-25) | 0,5% |
| Erfindungsgemäßes Beispiel 1 | 2,50% |
| TEGO^{Ⓡ}Alkanol 1618, Evonik Industries AG (INCI: CetearylAlcohol) | 5,00% |
| Preservative, Perfume | q.s. |

**Formulierungsbeispiel 3) Rinse-Off Conditioner**

| | |
|---|---|
| Water | 91,0% |
| Erfindungsgemäßes Beispiel 1 | 2,00% |
| VARISOFT^{Ⓡ} BT 85, Evonik Industries AG (INCI: Behentrimonium Chloride) | 2,00% |
| TEGO^{Ⓡ}Alkanol 1618, Evonik Industries AG (INCI: CetearylAlcohol) | 5,00% |
| Preservative, Perfume | q.s. |

**Formulierungsbeispiel 4) Rinse-Off Conditioner**

| | |
|---|---|
| Water | 90,20% |
| Erfindungsgemäßes Beispiel 1 | 2,00% |
| VARISOFT^{Ⓡ} EQ 65, Evonik Industries AG (INCI: DistearoylDimonium Chloride, Cetearyl Alcohol) | 2,00% |
| TEGO^{Ⓡ}Alkanol 1618, Evonik Industries AG (INCI: CetearylAlcohol) | 5,80% |
| Preservative, Perfume | q.s. |

**Formulierungsbeispiel 5) Rinse-Off Conditioner**

| | |
|---|---|
| Water | 89,20% |
| TEGINACID^{®} C, Evonik Industries AG (INCI: Ceteareth-25) | 0,5% |
| VARlSOFT^{Ⓡ} EQ 65, Evonik Industries AG (INCI: DistearoylDimonium Chloride, Cetearyl Alcohol) | 2,00% |
| Erfindungsgemäßes Beispiel 1 | 2,00% |
| ABIL^{Ⓡ} Quat 3272, Evonik Industries AG (INCI: Quaternium-80) | 1,30% |
| TEGO^{Ⓡ}Alkanol 1618, Evonik Industries AG (INCI: CetearylAlcohol) | 5,00% |
| Preservative, Perfume | q.s. |

**Formulierungsbeispiel 6) Rinse-Off Conditioner**

| | |
|---|---|
| TEGINACID^{Ⓡ} C, Evonik Industries AG (INCI: Ceteareth-25) | 0,50% |
| TEGO^{Ⓡ}Alkanol 16, Evonik Industries AG (INCI: CetylAlcohol) | 2,00% |
| TEGO^{Ⓡ} Amid S 18, Evonik Industries AG (INCI: Stearamidopropyl Dimethylamine) | 1,00% |
| Erfindungsgemäßes Beispiel 1 | 1,50% |
| PropyleneGlycol | 2,00% |
| CitricAcid Monohydrate | 0,30% |
| Water | 92,70% |
| Preservative, Perfume | q.s. |

**Formulierungsbeispiel 7) Rinse-Off Conditioner**

| | |
|---|---|
| TEGINACID^{Ⓡ} C, Evonik Industries AG (INCI: Ceteareth-25) | 0,50% |
| TEGO^{Ⓡ}Alkanol 16, Evonik Industries AG (INCI: CetylAlcohol) | 5,00% |
| TEGOSOFT^{Ⓡ} DEC, Evonik Industries AG (INCI: Diethylhexyl Carbonate) | 1,00% |
| Erfindungsgemäßes Beispiel 1 | 1,50% |
| Water | 89,20% |
| TEGO^{Ⓡ} Cosmo C 100 Evonik Industries AG (INCI: Creatine) | 0,50% |
| PropyleneGlycol | 2,00% |
| CitricAcid Monohydrate | 0,30% |
| Preservative, Perfume | q.s. |

**Formulierungsbeispiel 8) Leave-In Conditioner Spray**

| | |
|---|---|
| Lactic Acid, 80% | 0,40% |
| Water | 95,30% |
| Erfindungsgemäßes Beispiel 1 | 1,20% |
| TEGIN^{Ⓡ} G 1100 Pellets, Evonik Industries AG (INCI: GlycolDistearate) | 0,60% |
| TEGO^{Ⓡ} Care PS, Evonik Industries AG (INCI: Methyl Glucose Sesquistearate) | 1,20% |
| TEGOSOFT^{Ⓡ} DEC, Evonik Industries AG (INCI: Diethylhexyl Carbonate) | 1,30% |
| Preservative, Perfume | q.s. |

**Formulierungsbeispiel 9) Leave-In Conditioner Spray**

| | |
|---|---|
| Lactic Acid, 80% | 0,40% |
| Water | 95,30% |
| TEGO^{Ⓡ} Amid S 18, Evonik Industries AG (INCI: StearamidopropylDimethylamine) | 1,20% |
| Erfindungsgemäßes Beispiel 1 | 0,30% |
| TEGIN^{Ⓡ} G 1100 Pellets, Evonik Industries AG (INCI: GlycolDistearate) | 0,90% |
| TEGO^{Ⓡ} Care PS, Evonik Industries AG (INCI: Methyl Glucose Sesquistearate) | 1,60% |
| TEGOSOFT^{Ⓡ} DEC, Evonik Industries AG (INCI: Diethylhexyl Carbonate) | 0,30% |
| Preservative, Perfume | q.s. |

**Formulierungsbeispiel 10)Leave-ln Conditioner Spray**

| | |
|---|---|
| TAGAT^{Ⓡ} CH-40, Evonik Industries AG (INCI: PEG-40 Hydrogenated Castor Oil) | 2,20% |
| Ceramide VI, Evoniklndustries AG (INCI: Ceramide 6 II) | 0,05% |
| Perfume | 0,20% |
| Water | 90,95% |
| Erfindungsgemäßes Beispiel 1 | 0,30% |
| LACTIL^{Ⓡ} Evonik Industries AG (INCI: Sodium Lactate; Sodium PCA; Glycine; Fructose; Urea; Niacinamide; Inositol; Sodium benzoate; Lactic Acid) | 2,00% |
| TEGO^{Ⓡ}Betain F 50 Evonik Industries AG 38% (INCI: CocamidopropylBetaine) | 2,30% |
| Citric Acid (10% in water) | 2,00% |

**Formulierungsbeispiel 11)Leave-ln ConditionerFoam**

| | |
|---|---|
| Erfindungsgemäßes Beispiel 1 | 0,30% |
| TAGAT^{Ⓡ} CH-40, Evonik Industries AG (INCI: PEG-40 Hydrogenated Castor Oil) | 1,0% |
| Perfume | 0,30% |
| TEGO^{Ⓡ}Betain 810, Evonik Industries AG (INCI: Capryl/CapramidopropylBetaine) | 2,00% |
| Water | 94,00% |
| TEGO^{Ⓡ} Cosmo C 100, Evonik Industries AG (INCI: Creatine) | 0,50% |
| TEGOCEL^{Ⓡ} HPM 50, Evonik Industries AG (INCI: Hydroxypropyl Methylcellulose) | 0,30% |
| VARISOFT^{Ⓡ} 300, Evonik Industries AG (INCI: Cetrimonium Chloride) | 1,0% |
| LACTIL^{Ⓡ} Evonik Industries AG (INCI: Sodium Lactate; Sodium PCA; Glycine; Fructose; Urea; Niacinamide; Inositol; Sodium benzoate; Lactic Acid) | 0,50% |
| Citric Acid, 30% | 0,10% |
| Preservative | q.s. |

**Formulierungsbeispiel 12) Strong Hold Styling Gel**

| | |
|---|---|
| TEGO^{Ⓡ}Carbomer 141, Evonik Industries AG (INCI: Carbomer) | 1,20% |
| Water | 66,70% |
| NaOH, 25% | 2,70% |
| PVP/VA W-735, ISP (INCI: PVP/VA Copolymer | 16,00% |
| Erfindungsgemäßes Beispiel 1 | 0,30% |
| AlcoholDenat. | 10,50% |
| TAGAT^{Ⓡ} O 2 V, Evonik Industries AG (INCI: PEG-20 Glyceryl Oleate) | 2,00% |
| Perfume | 0,30% |
| ABIL^{Ⓡ} B 88183, Evonik Industries AG (INCI: PEG/PPG-20/6 Dimethicone) | 0,30% |
| Preservative | q.s. |

**Formulierungsbeispiel 13) Körperpflegemittel**

| | |
|---|---|
| TEXAPON^{Ⓡ} NSO, Cognis, 28%-ig (INCI: SodiumLaureth Sulfate) | 30,00% |
| TEGOSOFT^{Ⓡ} PC 31, Evonik Industries AG (INCI: Polyglyceryl-3 Caprate) | 0,70% |
| Erfindungsgemäßes Beispiel 1 | 0,30% |
| Perfume | 0,30% |
| Water | 53,90% |
| TEGOCEL^{Ⓡ} HPM 4000, Evonik Industries AG (INCI: Hydroxypropyl Methylcellulose) | 0,30% |
| REWOTERlC^{Ⓡ} AM C, Evonik Industries AG, 32%-ig (INCI: SodiumCocoamphoacetate) | 10,00% |
| CitricAcid Monohydrate | 0,50% |
| REWODERM^{Ⓡ} LI S 80, Evonik Industries AG (INCI: PEG-200 HydrogenatedGlycerylPalmate; PEG-7 GlycerylCocoate) | 2,00% |
| TEGO^{Ⓡ} Pearl N 300, Evonik Industries AG (INCI: Glycol Distearate; Laureth-4; CocamidopropylBetaine) | 2,00% |

**Formulierungsbeispiel 14) Mild Foam Bath**

| | |
|---|---|
| TEXAPON^{Ⓡ} NSO, Cognis, 28%-ig (INCI: Sodium LaurethSulfate) | 27,00% |
| REWOPOL^{Ⓡ} SB FA 30, Evonik Industries AG, 40%-ig (INCI: DisodiumLaurethSulfosuccinate) | 12,00% |
| TEGOSOFT^{Ⓡ} LSE 65 K SOFT, Evonik Industries AG (INCI: Sucrose Cocoate) | 2,00% |
| Water | 39,00% |
| REWOTERIC^{Ⓡ} AM C, Evonik Industries AG, 32%-ig (INCI: SodiumCocoamphoacetate) | 13,00% |
| Erfindungsgemäßes Beispiel 1 | 0,40% |
| Citric Acid (30% in water) | 3,00% |
| ANTlL^{Ⓡ} 171 Evonik Industries AG (INCI: PEG-18 GlycerylOleate/Cocoate) | 1,60% |
| TEGO^{Ⓡ} Pearl N 300 Evonik Industries AG (INCI: Glycol Distearate; Laureth-4; CocamidopropylBetaine) | 2,00% |

**Formulierungsbeispiel 15) Rinse-Off Conditioner**

| | |
|---|---|
| Water | 89,20% |
| Erfindungsgemäßes Beispiel 1 | 3,00% |
| ABIL^{Ⓡ} OSW 5, Evonik Industries AG (INCI: Cyclopentasiloxane; Dimethiconol) | 1,80% |
| TEGO^{Ⓡ}Alkanol 1618, Evonik Industries AG (INCI: CetearylAlcohol) | 6,00% |
| Preservative, Perfume | q.s. |

**Formulierunsbeisiel 16) Rinse-Off Conditioner**

| | |
|---|---|
| Water | 89,20% |
| TEGlNAClD^{®} C, Evonik Industries AG (INCI: Ceteareth-25) | 0,5% |
| VARlSOFT^{Ⓡ} EQ 65, Evonik Industries AG (INCI: DistearylDimonium Chloride, Cetearyl Alcohol) | 1,50% |
| Erfindungsgemäßes Beispiel 1 | 2,00% |
| ABIL^{Ⓡ} Soft AF 100, Evonik Industries AG (INCI: Methoxy PEG/PPG-7/3 AminopropylDimethicone) | 1,00% |
| TEGO^{Ⓡ}Alkanol 1618, Evonik Industries AG (INCI: CetearylAlcohol) | 5,80% |
| Preservative, Perfume | q.s. |

**Formulierungsbeispiel 17) Rinse-Off Conditioner**

| | |
|---|---|
| Water | 91,50% |
| TEGlNAClD^{®} C, Evonik Industries AG (INCI: Ceteareth-25) | 0,5% |
| Erfindungsgemäßes Beispiel 1 | 2,00% |
| SF 1708, Momentive (INCI: Amodimethicone) | 1,00% |
| TEGO^{Ⓡ}Alkanol 1618, Evonik Industries AG (INCI: CetearylAlcohol) | 5,00% |
| Preservative, Perfume | q.s. |

**Formulierunsbeisiel 18) 2in1 Shampoo & Intensive Conditioner**

| | |
|---|---|
| Water | 28,00% |
| Jaguar C-162 (HydroxypropylGuarHydroxypropyltimonium Chloride) | 0,5% |
| VARISOFT^{Ⓡ}BT 85, Evonik Industries AG (INCI: Behentrimonium Chloride) | 0,50% |
| Erfindungsgemäßes Beispiel 1 | 0,50% |
| TEGO^{Ⓡ}Alkanol 1618, Evonik Industries AG (INCI: CetearylAlcohol) | 1,00% |
| TEGlN^{Ⓡ} G 1100 Pellets, Evonik Industries AG (INCI: GlycolDistearate) | 0,80% |
| SodiumLaureth Sulfate, 28% | 39,0% |
| Petrolatum | 1,0% |
| ABIL^{Ⓡ}T- Quat 60, Evonik Industries AG (INCI: Silicone Quaternium-22) | 1,3% |
| REWOTERIC^{Ⓡ} AM C, Evonik Industries AG (INCI: SodiumCocoamphoacetate | 17,50% |
| CitricAcid (20%) | 6,3% |
| ANTlL^{Ⓡ} SPA 80, Evonik Industries AG ( INCI: Isostearamide MIPA; GlycerylLaurate) | 2,20% |
| NaCl | 1,0% |
| REWODERM^{Ⓡ} LI S 80, Evonik Industries AG (INCI: PEG- 200 HydrogenatedGlycerylPalmate; PEG-7 GlycerylCocoate) | 1,0% |
| Preservative, Perfume | q.s. |

**Formulierungsbeispiel 19: Pet Care - Conditioner**

| | |
|---|---|
| TEGO^{®}Alkanol 1618, Evonik Industries AG (INCI: CetearylAlcohol) | 4,0% |
| TEGINACID^{®} C, Evonik Industries AG (INCI: Ceteareth-25) | 0,5% |
| VARISOFT^{Ⓡ}BT 85, Evonik Industries AG (INCI: Behentrimonium Chloride) | 2,0% |
| Erfindungsgemäßes Beispiel 1 | 1,0% |
| TEGlN^{Ⓡ} M, Evonik Industries AG (INCI: GlycerylStearate) | 0,5% |
| Water | 93,0% |
| Preservative, Perfume | q.s. |

**Formulierungsbeispiel 20: In- Shower Hair and Body Conditioner (O/W emulsion)**

| | |
|---|---|
| ABIL^{Ⓡ} Soft AF 100, Evonik Industries AG (INCI: Methoxy PEG/PPG-7/3 AminopropylDimethicone) | 0,5% |
| Erfindungsgemäßes Beispiel 1 | 1,0% |
| VARISOFT^{Ⓡ}BT 85, Evonik Industries AG (INCI: Behentrimonium Chloride) | 0,5% |
| TEGO^{Ⓡ}Alkanol 16, Evonik Industries AG (INCI: CetylAlcohol) | 2,0% |
| SimmondsiaChinensis (Jojoba) Seed Oil | 0,5% |
| Water | 93,5% |
| Glycerin | 2,0% |
| Panthenol | 0,2% |
| Preservative, Perfume | q.s. |

**Formulierungsbeispiel 21: Hot Oil Treatment**

| | |
|---|---|
| Water | 96,5 |
| Polyquaternium- 10 | 1,0% |
| Hydroxyethylcellulose | 0,5% |
| Erfindungsgemäßes Beispiel 1 | 1,0% |
| VARISOFT^{Ⓡ}BT 85, Evonik Industries AG (INCI: Behentrimonium Chloride) | 1,0% |
| Lauramide DEA | 1,0% |
| CitricAcid, 30% | q.s. |
| Preservative, Perfume | q.s. |

**Formulierungsbeispiel 22: Strong conditioning hair rinse for damaged hair**

| | |
|---|---|
| VARISOFT^{Ⓡ}BT 85, Evonik Industries AG (INCI: Behentrimonium Chloride) | 0,5% |
| Erfindungsgemäßes Beispiel 1 | 0,5% |
| COSMOFERM^{Ⓡ}Mix III, Evonik Industries AG (INCI: IsocetylAlcohol; Ceramide NP; CetylAlcohol) | 2,0% |
| TEGINACID^{®} C, Evonik Industries AG (INCI: Ceteareth- 25) | 0,5% |
| ABlL^{Ⓡ} Soft AF 100, Evonik Industries AG (INCI: Methoxy PEG/PPG-7/3 AminopropylDimethicone) | 0,3% |
| TEGO^{Ⓡ}Alkanol 16, Evonik Industries AG (INCI: CetylAlcohol) | 2,0% |
| TEGIN^{Ⓡ} M, Evonik Industries AG (INCI: GlycerylStearate) | 1,0% |
| ABlL^{Ⓡ} OSW 5, Evonik Industries AG (INCI: Cyclopentasiloxane; Dimethiconol) | 7,5% |
| Water | 86,2% |
| Preservative, Perfume | q.s. |

**Formulierungsbeispiel 23: Conditioning hair rinse for coloured hair**

| | |
|---|---|
| TEGlN^{Ⓡ} M, Evonik Industries AG (INCI: GlycerylStearate) | 2,0% |
| TEGO^{®}Alkanol 1618, Evonik Industries AG (INCI: CetearylAlcohol) | 3,0% |
| TEGO^{®}Amid S 18, Evonik Industries AG (INCI: StearamidopropylDimethylamine) | 0,7% |
| VARISOFT^{®}BT 85, Evonik Industries AG (INCI: Behentrimonium Chloride) | 0,8% |
| Erfindungsgemäßes Beispiel 1 | 0,8% |
| VARISOFT^{®}BW 575 PG, Evonik Industries AG (INCI: Quaternium-87) | 0,9% |
| Water | 90,9% |
| 1,2- Propylenglykol (PropyleneGlycol) | 1,0% |
| CitricAcid (20%) | 0,7% |
| Preservative, Perfume | q.s. |

**Formulierungsbeispiel 24: Conditioning hair rinse**

| | |
|---|---|
| TEGO^{®}Alkanol 1618, Evonik Industries AG (INCI: Cetearyl Alcohol) | 5,0% |
| TEGO^{®}Care 450 Evonik Industries AG (INCI: Polyglyceryl-3 Methylglucose Distearate) | 1,0% |
| Erfindungsgemäßes Beispiel 1 | 1,0% |
| Water | 92,55% |
| Preservative | 0,45% |

**Formulierungsbeispiel 25: Conditioning hair rinse**

| | |
|---|---|
| TEGO^{®}Alkanol 1618, Evonik Industries AG (INCI: Cetearyl Alcohol) | 5,0% |
| TEGO^{Ⓡ} Care CG 90, Evonik Industries AG (INCI: Cetearyl Glucoside) | 1,0% |
| Erfindungsgemäßes Beispiel 1 | 1,0% |
| Water | 92,55% |
| Preservative | 0,45% |

**Formulierungsbeispiel 26: Conditioning hair rinse**

| | |
|---|---|
| TEGO^{®}Alkanol 1618, Evonik Industries AG (INCI: Cetearyl Alcohol) | 5,0% |
| TEGO^{Ⓡ} Care PSC 3, Evonik Industries AG (INCI: Polyglyceryl-3 Dicitrate / Stearate) | 1,0% |
| Erfindungsgemäßes Beispiel 1 | 1,0% |
| Water | 92,55% |
| Preservative | 0,45% |

**Formulierungsbeispiel 27: Conditioning hair rinse**

| | |
|---|---|
| TEGO^{®}Alkanol 1618, Evonik Industries AG (INCI: Cetearyl Alcohol) | 5,0% |
| TEGO^{Ⓡ} Acid S 40 P, Evonik Industries AG (INCI: PEG-40 Stearate) | 1,0% |
| Erfindungsgemäßes Beispiel 1 | 1,0% |
| Water | 92,55% |
| Preservative | 0,45% |

**Formulierungsbeispiel 28: Conditioning hair rinse**

| | |
|---|---|
| TEGO^{®}Alkanol 1618, Evonik Industries AG (INCI: Cetearyl Alcohol) | 5,0% |
| ABlL^{Ⓡ} Care 85, Evonik Industries AG (INCI: Bis-PEG/PPG-16/16 PEG/PPG-16/16 Dimethicone; Caprylic/Capric Triglyceride) | 1,0% |
| Erfindungsgemäßes Beispiel 1 | 1,0% |
| Water | 92,55% |
| Preservative | 0,45% |

**Formulierungsbeispiel 29: Conditioning hair rinse**

| | |
|---|---|
| TEGO^{®} Alkanol 1618, Evonik Industries AG (INCI: Cetearyl Alcohol) | 5,0% |
| ABlL^{Ⓡ} Care XL 80, Evonik Industries AG (INCI: Bis-PEG/PPG-20/5 PEG/PPG-20/5 Dimethicone; Methoxy PEG/PPG-25/ 4 Dimethicone; Caprylic/Capric Triglyceride) | 1,0% |
| Erfindungsgemäßes Beispiel 1 | 1,0% |
| Water | 92,55% |
| Preservative | 0,45% |

**Formulierungsbeispiel 30: Conditioning hair rinse**

| | |
|---|---|
| TEGO^{®}Alkanol 1618, Evonik Industries AG (INCI: Cetearyl Alcohol) | 5,0% |
| TEGO^{®} Alkanol CS 20 P, Evonik Industries AG (INCI: Ceteareth-20) | 0,5% |
| VARlSOFT^{Ⓡ} EQ 65, Evonik Industries AG (INCI: Distearoylethyl Dimonium Chloride; Cetearyl Alcohol) | 2,0% |
| Erfindungsgemäßes Beispiel 1 | 1,3% |
| ABlL^{Ⓡ} Quat 3474, Evonik Industries AG (INCI: Quaternium-80) | 0,5% |
| EDTA | 0,02 |
| Water | 90,68% |
| Preservative, Perfume | q.s. |

**Formulierungsbeispiel 31: Conditioning hair rinse**

| | |
|---|---|
| TEGO^{®}Alkanol 1618, Evonik Industries AG (INCI: Cetearyl Alcohol) | 5,0% |
| TEGO^{®} Alkanol CS 20 P, Evonik Industries AG (INCI: Ceteareth-20) | 0,5% |
| VARlSOFT^{Ⓡ} BT 85, Evonik Industries AG (INCI: Behentrimonium Chloride) | 2,0% |
| Erfindungsgemäßes Beispiel 1 | 1,3% |
| ABlL^{Ⓡ} Quat 3474, Evonik Industries AG (INCI: Quaternium-80) | 0,5% |
| EDTA | 0,02 |
| Water | 90,68% |
| Preservative, Perfume | q.s. |

**Formulierungsbeispiel 32: Conditioning hair rinse**

| | |
|---|---|
| TEGO^{®}Alkanol 1618, Evonik Industries AG (INCI: Cetearyl Alcohol) | 7,0% |
| TEGINACID^{®} C, Evonik Industries AG (INCI: Ceteareth-25) | 1,0% |
| Erfindungsgemäßes Beispiel 1 | 1,0% |
| Water | 91,0% |
| Preservative, Perfume | q.s. |

**Formulierungsbeispiel 33: Conditioning hair rinse**

| | |
|---|---|
| TEGO^{®}Alkanol 1618, Evonik Industries AG (INCI: Cetearyl Alcohol) | 7,0% |
| TEGINACID^{®} C, Evonik Industries AG (INCI: Ceteareth-25) | 1,0% |
| Erfindungsgemäßes Beispiel 1 | 1,0% |
| ABIL^{Ⓡ} ME 45, Evonik Industries AG (INCI: Silicone Quaternium-22; Polyglyceryl-3 Caprate; Dipropylene Glycol; Cocamidopropyl Betaine) | 1,7% |
| Water | 89,3% |
| Preservative, Perfume | q.s. |

**Formulierungsbeispiel 34: Conditioning hair rinse**

| | |
|---|---|
| TEGO^{®}Alkanol 1618, Evonik Industries AG (INCI: Cetearyl Alcohol) | 7,0% |
| TEGINACID^{®} C, Evonik Industries AG (INCI: Ceteareth-25) | 1,0% |
| Erfindungsgemäßes Beispiel 1 | 1,0% |
| Amodimethicone | 1,0% |
| Water | 90,0% |
| Preservative, Perfume | q.s. |

**Formulierungsbeispiel 35: Conditioning hair rinse**

| | |
|---|---|
| TEGO^{®}Alkanol 1618, Evonik Industries AG (INCI: Cetearyl Alcohol) | 7,0% |
| TEGINACID^{®} C, Evonik Industries AG (INCI: Ceteareth-25) | 1,0% |
| Erfindungsgemäßes Beispiel 1 | 1,0% |
| ABIL^{Ⓡ} Soft AF 300, Evonik Industries AG (INCI: Aminopropyl Dimethicone ) | 1,0% |
| Water | 90,0% |
| Preservative, Perfume | q.s. |

**Formulierungsbeispiel 36: Conditioning hair rinse**

| | |
|---|---|
| TEGO^{®}Alkanol 16, Evonik Industries AG (INCI: Cetyl Alcohol) | 5,0% |
| TEGINACID^{®} C, Evonik Industries AG (INCI: Ceteareth-25) | 0,5% |
| Erfindungsgemäßes Beispiel 1 | 1,0% |
| Water | 93,5% |
| Preservative, Perfume | q.s. |

**Formulierungsbeispiel 37: Conditioning hair rinse**

| | |
|---|---|
| TEGO^{®}Alkanol 16, Evonik Industries AG (INCI: Cetyl Alcohol) | 5,0% |
| TEGINACID^{®} C, Evonik Industries AG (INCI: Ceteareth-25) | 0,5% |
| TEGO^{®}Amid S 18, Evonik Industries AG (INCI: Stearamidopropyl Dimethylamine) | 0,5% |
| Erfindungsgemäßes Beispiel 1 | 0,5% |
| Water | 93,5% |
| Preservative, Perfume | q.s. |

**Formulierungsbeispiel 38: Conditioning hair rinse**

| | |
|---|---|
| TEGO^{®}Alkanol 18, Evonik Industries AG (INCI: Stearyl Alcohol) | 5,0% |
| TEGINACID^{®} C, Evonik Industries AG (INCI: Ceteareth-25) | 0,5% |
| TEGO^{®}Amid S 18, Evonik Industries AG (INCI: Stearamidopropyl Dimethylamine) | 0,5% |
| Erfindungsgemäßes Beispiel 1 | 0,5% |
| Water | 93,5% |
| Preservative, Perfume | q.s. |

**Formulierungsbeispiel 39: Conditioning hair rinse**

| | |
|---|---|
| TEGO^{®}Alkanol 18, Evonik Industries AG (INCI: Stearyl Alcohol) | 5,0% |
| TEGINACID^{®} C, Evonik Industries AG (INCI: Ceteareth-25) | 0,5% |
| Erfindungsgemäßes Beispiel 1 | 1,0% |
| Water | 93,5% |
| Preservative, Perfume | q.s. |

**Formulierungsbeispiel 40: Conditioning hair rinse**

| | |
|---|---|
| TEGO^{®}Alkanol 1618, Evonik Industries AG (INCI: Cetearyl Alcohol) | 7,0% |
| TEGO^{®} Alkanol S 20 P, Evonik Industries AG (INCI: Steareth-20) | 1,0% |
| Erfindungsgemäßes Beispiel 1 | 1,0% |
| Water | 91,0% |
| Preservative, Perfume | q.s. |

**Formulierungsbeispiel 41: Conditioning hair rinse**

| | |
|---|---|
| TEGO^{®}Alkanol 1618, Evonik Industries AG (INCI: | 7,0% |
| Cetearyl Alcohol) | |
| TEGO^{®} Alkanol L 23 P, Evonik Industries AG (INCI: Laureth-23) | 1,0% |
| Erfindungsgemäßes Beispiel 1 | 1,0% |
| Water | 91,0% |
| Preservative, Perfume | q.s. |

## Patentansprüche

1. Kosmetische Formulierung enthaltend
0,2 bis 25 Gew.-% mindestens einer Verbindung der allgemeinen Formel I) 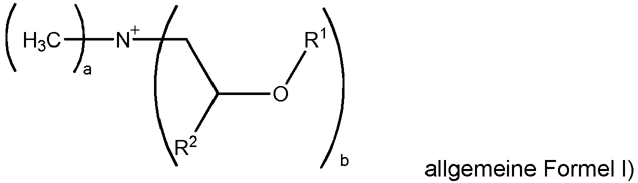
mit R¹ ein Acylrest einer mindestens einfach ungesättigten Fettsäure mit einer Kettenlänge von 18 bis 24 Kohlenstoffatomen oder ein Acylrest der Isostearinsäure oder Rizinolsäure,
mit R² ein Alkylrest mit 1 bis 6 Kohlenstoffatomen,
wobei a = 1 bis 3 und b = 1 bis 3,
mit der Maßgabe, dass a + b = 4,
40 bis 99,8 Gew.-% Wasser und gegebenenfalls
0 bis 8 Gew.-% eine Verbindung der allgemeinen Formel Ia) 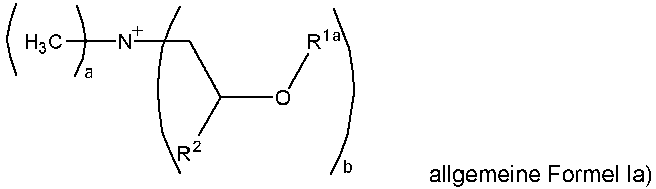
mit R^{1a} ein Acylrest einer anderen Carbonsäure als für R¹ definiert und
mit R² ein Alkylrest mit 1 bis 6 Kohlenstoffatomen,
wobei a = 1 bis 3 und b = 1 bis 3,
mit der Maßgabe, dass a + b = 4,
wobei sich die Gewichtsprozente auf die Gesamtformulierung beziehen, **dadurch gekennzeichnet, dass**
die Verbindungen der allgemeinen Formel I) mindestens 50 Gew.-%, bezogen auf alle in der Formulierung enthaltene Verbindungen der allgemeinen Formel I) und Ia) ausmachen,
und **dadurch gekennzeichnet, dass**
die Verbindungen der allgemeinen Formel I)
mit R¹ ein Acylrest einer mindestens einfach ungesättigten Fettsäure mit einer Kettenlänge von 18 bis 24 Kohlenstoffatomen ausgewählt aus den Acylresten der Säuren aus der Gruppe Ölsäure, Linolsäure, Alpha-Linolensäure und Gamma-Linolensäure, oder ein Acylrest der Isostearinsäure oder der Rizinolsäure
mindestens 50 Gew.-%, bezogen auf alle in der Formulierung enthaltene Verbindungen der allgemeinen Formel I) und Ia) ausmachen.

2. Formulierung gemäß Anspruch 1, **dadurch gekennzeichnet, dass** a = b = 2.

3. Formulierung gemäß mindestens einem der vorherigen Ansprüche, **dadurch gekennzeichnet, dass** sie keine Fettsäuren oder Fettsäuresalze enthält.

4. Formulierung gemäß mindestens einem der vorherigen Ansprüche zusätzlich enthaltend
0,5 bis 20 Gew.-% mindestens eines Fettalkohols,
wobei sich die Gewichtsprozente auf die Gesamtformulierung beziehen.

5. Formulierung gemäß Anspruch 4, **dadurch gekennzeichnet, dass** der Fettalkohol ausgewählt ist aus der Gruppe umfassend Octanol, Decanol, Laurylalkohol, Isolaurylalkohol, Anteisolaurylalkohol, Myristylalkohol, Isomyristylalkohol, Cetylalkohol, Palmoleylalkohol, Stearylalkohol, Isostearylalkohol, Anteisostearylalkohol, Eicosanol, Petroselinylalkohol, Guerbetalkohol, Arachylalkohol, Gadoleylalkohol, Behenylalkohol, Erucylalkohol, Heptacosanol, Octacosanol, und Melissylalkohol sowie Mischungen davon.

6. Formulierung gemäß mindestens einem der vorherigen Ansprüche zusätzlich enthaltend mindestens ein Gegenanion zu der Verbindung der allgemeinen Formel I) ausgewählt aus der Gruppe umfassend Chlorid, Bromid, lodid, Alkylsulfat, z.B. Methylsulfat, Ethylsulfat, Alkylsulfonat, z.B. Methylsulfonat, Triflat, Tosylat, Phosphat, Sulfat, Hydrogensulfat, Lactat, Glycolat, Acetat und Citrat.

7. Verbindung der allgemeinen Formel I) 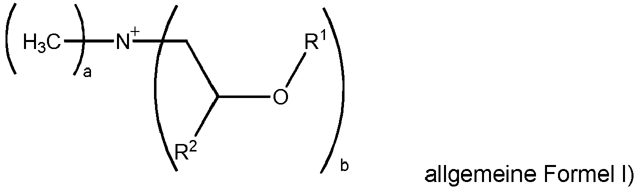
mit R¹ ein Acylrest der Isostearinsäure,
mit R² ein Alkylrest mit 1 bis 6 Kohlenstoffatomen, bevorzugt Methyl, Ethyl, Propyl, Isopropyl, besonders bevorzugt Methyl,
wobei a = 1 bis 3 und b = 1 bis 3
mit der Maßgabe, dass a + b =4.

8. Verwendung einer Verbindung der allgemeinen Formel I) 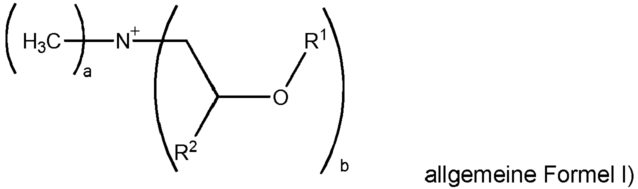
mit R¹ ein Acylrest der Isostearinsäure,
mit R² ein Alkylrest mit 1 bis 6 Kohlenstoffatomen,
wobei a = 1 bis 3 und b = 1 bis 3,
mit der Maßgabe, dass a + b =4
oder einer Formulierung gemäß mindestens einem der Ansprüche 1 bis 6 zur kosmetischen Behandlung von keratinischen Fasern.

9. Verwendung gemäß Anspruch 8 zur Erhöhung des Glanzes der Faser.

10. Verwendung gemäß Anspruch 8 zur Erhöhung der Flexibilität der Faser.

11. Verwendung gemäß Anspruch 8 zur Erhöhung der Kämmbarkeit der Faser.

12. Verwendung gemäß Anspruch 8 zur Erhöhung der Spannkraft der Faser.

13. Verwendung gemäß Anspruch 8 zur Reduktion der antistatischen Kräfte zwischen den Fasern.

14. Verwendung gemäß Anspruch 8 zum Schutz der Fasern vor Hitze.

## Claims

1. Cosmetic formulation comprising
0.2 to 25% by weight of at least one compound of the general formula I) 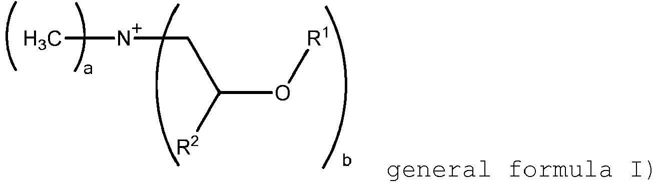 where R¹ is an acyl radical of an at least monounsaturated fatty acid with a chain length of 18 to 24 carbon atoms or an acyl radical of isostearic acid or ricinoleic acid,
where R² is an alkyl radical having 1 to 6 carbon atoms,
where a = 1 to 3 and b = 1 to 3,
with the proviso that a + b = 4,
40 to 99.8% by weight water and optionally 0 to 8% by weight of a compound of the general formula Ia) 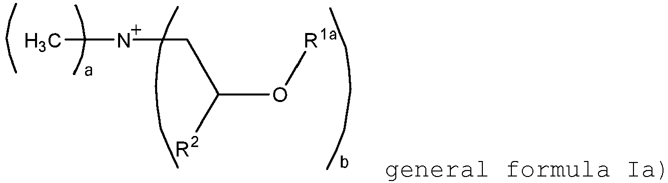 where R^{1a} is an acyl radical of another carboxylic acid as defined for R¹ and
where R² is an alkyl radical having 1 to 6 carbon atoms,
where a = 1 to 3 and b = 1 to 3,
with the proviso that a + b = 4,
where the percentages by weight refer to the total formulation,
**characterized in that**
the compounds of the general formula I) constitute at least 50% by weight, based on all of the compounds of the general formula I) and Ia) present in the formulation,
and **characterized in that**
the compounds of the general formula I)
where R¹ is an acyl radical of an at least monounsaturated fatty acid with a chain length of 18 to 24 carbon atoms selected from the acyl radicals of the acids from the group comprising oleic acid, linoleic acid, alpha-linolenic acid and gamma-linolenic acid, or an acyl radical of isostearic acid or ricinoleic acid constitute at least 50% by weight, based on all of the compounds of the general formula I) and Ia) present in the formulation.

2. Formulation according to Claim 1, **characterized in that** a = b = 2.

3. Formulation according to at least one of the preceding claims, **characterized in that** it comprises no fatty acids or fatty acid salts.

4. Formulation according to at least one of the preceding claims, additionally comprising 0.5 to 20% by weight of at least one fatty alcohol, where the percentages by weight refer to the total formulation.

5. Formulation according to Claim 4, **characterized in that** the fatty alcohol is selected from the group comprising octanol, decanol, lauryl alcohol, isolauryl alcohol, anteisolauryl alcohol, myristyl alcohol, isomyristyl alcohol, cetyl alcohol, palmoleyl alcohol, stearyl alcohol, isostearyl alcohol, anteisostearyl alcohol, eicosanol, petroselinyl alcohol, Guerbet alcohol, arachyl alcohol, gadoleyl alcohol, behenyl alcohol, erucyl alcohol, heptacosanol, octacosanol, and melissyl alcohol, and mixtures thereof.

6. Formulation according to at least one of the preceding claims, additionally comprising at least one counteranion to the compound of the general formula I) selected from the group comprising chloride, bromide, iodide, alkyl sulfate, e.g. methyl sulfate, ethyl sulfate, alkylsulfonate, e.g. methylsulfonate, triflate, tosylate, phosphate, sulfate, hydrogensulfate, lactate, glycolate, acetate and citrate.

7. Compound of the general formula I) 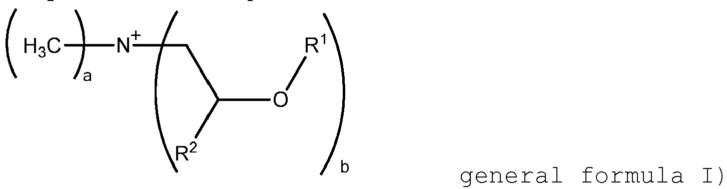 where R¹ is an acyl radical of isostearic acid,
where R² is an alkyl radical having 1 to 6 carbon atoms, preferably methyl, ethyl, propyl, isopropyl, particularly preferably methyl,
where a = 1 to 3 and b = 1 to 3
with the proviso that a + b = 4.

8. Use of a compound of the general formula I) 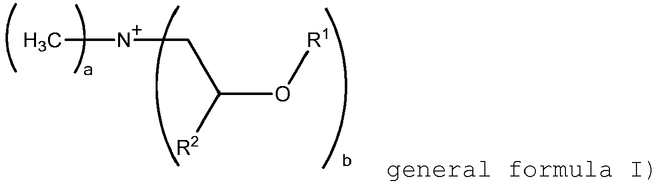 where R¹ is an acyl radical of isostearic acid,
where R² is an alkyl radical having 1 to 6 carbon atoms,
where a = 1 to 3 and b = 1 to 3,
with the proviso that a + b = 4
or of a formulation according to at least one of Claims 1 to 6
for the cosmetic treatment of keratin fibres.

9. Use according to Claim 8 for increasing the shine of the fibres.

10. Use according to Claim 8 for increasing the flexibility of the fibres.

11. Use according to Claim 8 for increasing the combability of the fibres.

12. Use according to Claim 8 for increasing the elasticity of the fibres.

13. Use according to Claim 8 for reducing the antistatic forces between the fibres.

14. Use according to Claim 8 for protecting the fibres against heat.

## Revendications

1. Formulation cosmétique contenant
0,2 à 25 % en poids d'au moins un composé de Formule générale I) 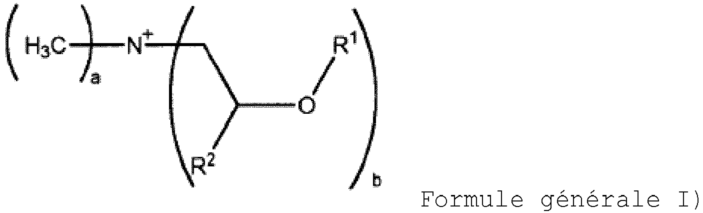
dans laquelle R¹ représente un radical acyle d'un acide gras au moins monoinsaturé, ayant une longueur de chaîne de 18 à 24 atomes de carbone, ou un radical acyle de l'acide isostéarique ou de l'acide ricinoléique,
R² représente un radical alkyle ayant 1 à 6 atomes de carbone,
a = 1 à 3 et b = 1 à 3,
à la condition que a + b = 4,
40 à 99,8 % en poids d'eau et éventuellement 0 à 8 % en poids d'un composé de Formule générale la) 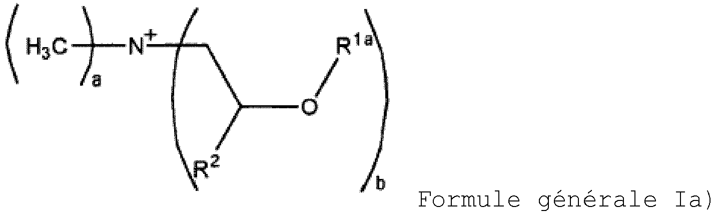 dans laquelle R^{1a} représente un radical acyle d'un autre acide carboxylique que celui défini pour R¹ et R² représente un radical alkyle ayant 1 à 6 atomes de carbone,
a = 1 à 3 et b = 1 à 3,
à la condition que a + b = 4,
les pourcentages en poids étant rapportés à la formulation totale,
**caractérisée en ce que**
les composés de Formule générale I) représentent au moins 50 % en poids, par rapport à la totalité des composés contenus dans la formulation de Formules générales I) et Ia),
et **caractérisée en ce que**
les composés de Formule générale I)
dans laquelle R¹ représente un radical acyle d'un acide gras au moins monoinsaturé ayant une longueur de chaîne de 18 à 24 atomes de carbone, choisi parmi les radicaux acyle des acides du groupe acide oléique, acide linoléique, acide alpha-linolénique et acide gamma-linolénique, ou un radical acyle de l'acide isostéarique ou de l'acide ricinoléique représentent au moins 50 % en poids, par rapport à la totalité des composés contenus dans la formulation de Formules générales I) et Ia).

2. Formulation selon la revendication 1, **caractérisée en ce que** a = b = 2.

3. Formulation selon au moins l'une des revendications précédentes, **caractérisée en ce qu'**elle ne contient ni acides gras, ni sels d'acides gras.

4. Formulation selon au moins l'une des revendications précédentes, contenant en outre 0,5 à 20 % en poids d'au moins un alcool gras, les pourcentages en poids étant rapportés à la formulation totale.

5. Formulation selon la revendication 4, **caractérisée en ce que** l'alcool gras est choisi dans le groupe comprenant l'octanol, le décanol, l'alcool laurylique, l'alcool isolaurylique, l'alcool antéisolaurylique, l'alcool myristylique, l'alcool isomyristylique, l'alcool cétylique, l'alcool palmoléylique, l'alcool stéarylique, l'alcool isostéarylique, l'alcool antéisostéarylique, l'éicosanol, l'alcool pétrosélinylique, l'alcool de Guerbet, l'alcool arachydilique, l'alcool gadoléylique, l'alcool béhénylique, l'alcool érucylique, l'heptacosanol, l'octacosanol et l'alcool mélissylique, ainsi que les mélanges de ceux-ci.

6. Formulation selon au moins l'une des revendications précédentes, contenant en outre un contre-anion par rapport au composé de Formule générale I) choisi dans le groupe comprenant les anions chlorure, bromure, iodure, alkylsulfate, par exemple méthylsulfate, éthylsulfate, alkylsulfonate, par exemple méthylsulfonate, triflate, tosylate, phosphate, sulfate, hydrogénosulfate, lactate, glycolate, acétate et citrate.

7. Composé de Formule générale I) 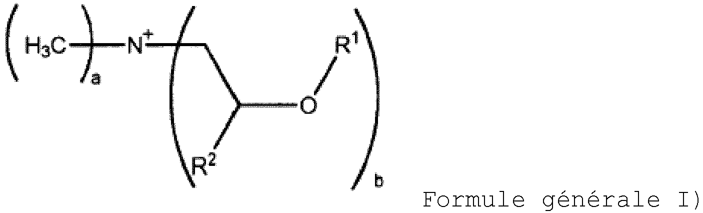
dans laquelle R¹ représente un radical acyle de l'acide isostéarique,
R² représente un radical alkyle ayant 1 à 6 atomes de carbone, de préférence méthyle, éthyle, propyle, isopropyle, d'une manière particulièrement préférée méthyle,
a = 1 à 3 et b = 1 à 3
à la condition que a + b = 4.

8. Utilisation d'un composé de Formule générale I) 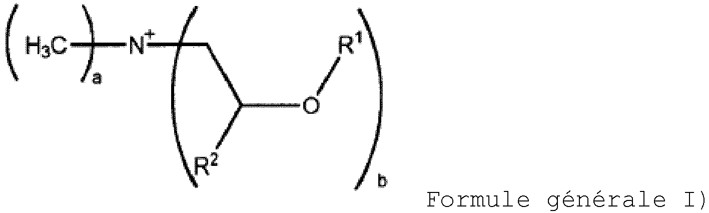
dans laquelle R¹ représente un radical acyle de l'acide isostéarique,
R² représente un radical alkyle ayant 1 à 6 atomes de carbone,
a = 1 à 3 et b = 1 à 3,
à la condition que a + b = 4
ou d'une formulation selon au moins l'une des revendications 1 à 6,
pour le traitement cosmétique de fibres kératiniques.

9. Utilisation selon la revendication 8 pour augmenter la brillance des fibres.

10. Utilisation selon la revendication 8 pour augmenter la souplesse des fibres.

11. Utilisation selon la revendication 8 pour augmenter la peignabilité des fibres.

12. Utilisation selon la revendication 8 pour augmenter la résilience des fibres.

13. Utilisation selon la revendication 8 pour réduire les forces antistatiques entre les fibres.

14. Utilisation selon la revendication 8 pour protéger les fibres de la chaleur.
